(19) Europäisches Patentamt
European Patent Office
Office européen des brevets

(11) **EP 2 409 700 A1**

(12) **EUROPEAN PATENT APPLICATION**

(43) Date of publication:
**25.01.2012 Bulletin 2012/04**

(51) Int Cl.:
***A61K 31/4188*** (2006.01)   ***A61P 35/00*** (2006.01)

(21) Application number: **11185843.7**

(22) Date of filing: **07.05.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **08.05.2007 US 916622 P**
**14.12.2007 US 13743 P**

(62) Document number(s) of the earlier application(s) in accordance with Art. 76 EPC:
**08754235.3 / 2 157 972**

(71) Applicant: **Schering Corporation**
**Kenilworth, NJ 07033-0530 (US)**

(72) Inventors:
• **Abutarif, Malaz**
**North Brunswick, NJ New Jersey 08902 (US)**
• **Statkevich, Paul**
**Raritan, NJ New Jersey 08869 (US)**

(74) Representative: **Simpson, Tobias Rutger et al**
**Mathys & Squire LLP**
**120 Holborn**
**London EC1N 2SQ (GB)**

Remarks:
This application was filed on 19-10-2011 as a divisional application to the application mentioned under INID code 62.

(54) **Methods of treatment using intravenous formulations comprising temozolomide**

(57) This invention relates to methods and drug products for treating proliferative disorders using intravenous formulations comprising temozolomide over a specific infusion time. These methods and drug products are particularly well-suited for patients who cannot swallow oral formulations. These methods and drug products also afford an added convenience to patients who are already receiving other therapeutic treatments.

FIG.2A

EP 2 409 700 A1

FIG.2B

**Description**

FIELD OF THE INVENTION

This invention relates to methods and drug products for treating proliferative disorders using intravenous formulations comprising temozolomide over a specific infusion time. These methods and drug products are particularly well-suited for patients who cannot swallow oral formulations. These methods and drug products also afford an added convenience to patients who are already receiving other therapeutic treatments.

BACKGROUND OF THE INVENTION

Brain tumors comprise approximately 2% of all malignant diseases. Stupp et al., J. Clin. Onc., 20(5):1375-1382 (2002). More than 17,000 cases are diagnosed every year in the United States, with approximately 13,000 associated deaths. The standard protocol for treating a malignant glioma involves cytoreduction through surgical resection, when feasible, followed by radiotherapy (RT) with or without adjuvant chemotherapy. Stupp et al., supra.

A chemotherapeutic agent approved for treating brain tumors is temozolomide or TMZ (marketed by Schering Corp. under the trade name of Temodar® in the United States and Temodal® in Europe). The chemical name for temozolomide is 3,4-dihydro-3-methyl-4-oxoimidazo[5,1-d]-as-tetrazine-8-carboxamide (see U.S. Pat. No. 5,260,291). Under biological conditions, temozolomide degrades by pH dependent hydrolysis to its active metabolite, MTIC (3-methyl-(triazen-1-yl)imidazole-4-carboxamide). The cytotoxicity of temozolomide or MTIC is thought to be primarily due to alkylation of DNA. Alkylation (methylation) occurs mainly at the $O^6$ and $N^7$ positions of guanine.

Temodar® Capsules are currently indicated in the United States for the treatment of adult patients with newly diagnosed gliobastoma multiforme, as well as refractory anaplastic astrocytoma, *i.e.*, patients at first relapse who have experienced disease progression on a drug regimen containing a nitrosourea and procarbazine. Temodal® Capsules are currently approved in Europe for the treatment of patients with malignant glioma, such as glioblastoma multiforme or anaplastic astrocytoma showing recurrence or progression after standard therapy.

As a result of disease progression or age, a subset of patients receiving TMZ treatment have difficulties in swallowing oral formulations. Swallowing difficulties can result in poor patient compliance and, in some cases, in patients being completely unable to take oral formulations, thereby producing a sub-optimal therapeutic benefit of the drug. Thus, there is an immediate need to develop intravenous (IV) formulations of TMZ, and methods of administering such formulations. Such a formulation would provide patients with swallowing difficulties an alternate route of administration. In addition, such a formulation would provide a significant convenience to patients who are already receiving other therapeutic agents.

In order to support the regulatory approval of an IV formulation of TMZ, it would be necessary to conduct either a large clinical efficacy study or bioequivalence studies between the oral (PO) and IV formulations. To establish bioequivalence, the pharmacokinetic (PK) profiles of both TMZ and MTIC following IV administration must match those following PO administration. In particular, the pharmacokinetic parameters Maximum Plasma Concentration (Cmax) and Total Area Under The Plasma Concentration-Time Curve (AUC) for TMZ and MTIC obtained following IV administration must match those obtained following PO administration with a 90 % confidence interval (*i.e.*, within a ratio of 80-125%). While the PK profiles of TMZ and MTIC for oral administration are known (see, e.g., Baker, Clinical Cancer Research, 5: 309-317 (1999), those for IV administration are not.

A factor relevant to establishing bioequivalence of the oral and IV formulations is TMZ's bioavailability. In general, the bioavailability of a drug administered orally can differ from the bioavailability of the same drug administered intravenously. This is because of the first-pass effect and /or incomplete absorption. For example, when a drug is administered orally, it is first absorbed within the gastrointestinal tract. The absorbed drug crosses the gastrointestinal tract membrane into cardiovascular veins, which, in turn, carry it to the liver and then the heart. The heart then distributes the drug into the systemic blood circulation. As the drug passes through the liver, it may be metabolized and a smaller portion of the absorbed drug will enter into systemic circulation, thereby reducing the drug's bioavailability. IV administration, on the other hand, injects the drug directly into systemic circulation and thus, avoids the first-pass effect.

While TMZ's oral bioavailability and PK profiles are important factors in establishing bioequivalence, they are not predictive of the IV PK profile. Thus, there is a significant need to determine the intravenous PK profiles of TMZ and MTIC. Such a determination would be useful in establishing bioequivalence and in supporting the regulatory approval of an IV formulation of TMZ.

SUMMARY OF THE INVENTION

It has now been found that TMZ exhibits high oral bioavailability in humans (96%; range: 89-100%; n = 13 patients). It has also been found that intravenous infusion of a formulation comprising TMZ over a period of about 0.6 hours to about 2.9 hours surprisingly matches the oral PK profiles of TMZ and MTIC.

Accordingly, the present invention provides methods of treating proliferative disorders using intravenous formulations comprising temozolomide over a specific infusion time. These methods may be used to treat patients having swallowing difficulties and/or receiving treatment with one or more additional therapeutic agents.

In some embodiments, the intravenous formulation is infused over a period of about 0.6 hours to about 2.9 hours. In some embodiments, the intravenous formulation is infused over a period of about 0.8 hours to about 2.5 hours. In some embodiments, the intravenous formulation is infused over a period of about 1 hour to about 2 hours. In some embodiments, the intravenous formulation is infused over a period of about 1 hour to about 1.75 hours. In some embodiments, the intravenous formulation is infused over a period of about 1.25 hours to about 1.75 hours. In other embodiments, the intravenous formulation is infused over a period of about 1.35 hours to about 1.65 hours. In other embodiments, the intravenous formulation is infused over a period of about 1.45 hours to about 1.55 hours. In other embodiments, the intravenous formulation is infused over a period of about 1.5 hours.

In some embodiments, the methods are used to treat patients having a proliferative disorder selected from carcinoma, sarcoma, glioma, glioblastoma, brain cancer, brain tumors, melanoma, lung cancer, thyroid follicular cancer, pancreatic cancer, anaplastic astrocytoma, bladder cancer, myelodysplasia, prostate cancer, testicular cancer, lymphoma, leukemia, mycosis fungoides, head and neck cancer, breast cancer, ovarian cancer, colorectal and/or colon cancer, or esophageal cancer. In some embodiments, the proliferative disorder is a brain tumor. In some aspects, the brain tumor is glioma. In some aspects, the glioma is anaplastic astrocytoma or glioblastoma multiforme. In other embodiments, the proliferative disorder is melanoma. In some embodiments, the proliferative disorder is lung cancer. In some aspects, the lung cancer is non-small cell lung cancer. In some embodiments, the proliferative disorder is carcinoma. In some embodiments, the proliferative disorder is sarcoma. In some embodiments, the proliferative disorder is brain cancer. In some embodiments, the proliferative disorder is thyroid follicular cancer. In some embodiments, the proliferative disorder is pancreatic cancer. In some embodiments, the proliferative disorder is bladder cancer. In some embodiments, the proliferative disorder is myelodysplasia. In some embodiments, the proliferative disorder is prostate cancer. In some embodiments, the proliferative disorder is testicular cancer. In some embodiments, the proliferative disorder is lymphoma. In some embodiments, the proliferative disorder is leukemia. In some embodiments, the proliferative disorder is mycosis fungoides. In some embodiments, the proliferative disorder is head and neck cancer. In some embodiments, the proliferative disorder is breast cancer. In some embodiments, the proliferative disorder is ovarian cancer. In some embodiments, the proliferative disorder is colorectal and/or colon cancer. In some embodiments, the proliferative disorder is esophageal cancer.

In some embodiments, the intravenous formulation comprises one or more of an aqueous diluent, dissolution enhancing agent, excipient, bulking agent, buffer or pH adjuster.

In some embodiments, the methods further comprise administering one or more additional therapeutic agents. In some embodiments, the one or more additional therapeutic agents are administered intravenously.

In some embodiments, the methods of treating proliferative disorders comprise administering the intravenous formulations according to a dosing regimen. In some embodiments, the dosing regimen is based on the condition to be treated. In some embodiments, the regimen is based upon the methylation state of the $O^6$-methylguanine-DNA transferase (MGMT) gene in a sample obtained from the patient. In other embodiments, the regimen is based upon the presence or absence of the MGMT protein in a sample obtained from the patient. In other embodiments, the regimen is based upon the level or enzymatic activity of the MGMT protein detected in a sample obtained from the patient.

In some embodiments, one or more of the above methods of treating proliferative disorders is described on the product information packaged with a pharmaceutical formulation comprising temozolomide, in which the formulation is designed for administration by intravenous infusion over a period of about 1 hour to about 2 hours, preferably over a period of about 1.25 to about 1.75 hours, preferably over a period of about 1.5 hours. The product information may also describe any of the temozolomide dosing regimens described above.

In a preferred embodiment, the invention comprises a method for treating a patient having a proliferative disorder comprising the step of administering to the patient a formulation comprising temozolomide or a pharmaceutically acceptable salt thereof, wherein the formulation is administered by intravenous infusion over a period of about I hour to about 2 hours, and wherein the administration of a single dose of 150 mg/m2 of the formulation achieves an arithmetic maximum plasma concentration (Cmax) of temozolomide in the range of about 5.5 to about 10.6 μg/mL and an arithmetic maximum plasma concentration (Cmax) of MTIC in the range of about 137 to about 916 ng/mL. In one embodiment, the arithmetic mean maximum plasma concentration (Cmax) of temozolomide is about 7.4 μg/mL, and the mean maximum plasma concentration (Cmax) of MTIC is about 320 ng/mL.

In another preferred embodiment, the invention comprises a method for treating a patient having a proliferative disorder comprising the step of administering to the patient in need thereof a formulation comprising temozolomide or a pharmaceutically acceptable salt thereof by intravenous infusion over a period of about 1 hour to about 2 hours, wherein a plot of the plasma concentration of temozolomide versus time following the administration of a single dose of 150 mg/m2 of the formulation yields an arithmetic AUC (from time zero to infinity) for temozolomide in the range of about 17.6 to about 37.0 (μg.hr)/mL, and a plot of the plasma concentration of MTIC versus time yields an arithmetic AUC (from time zero to infinity) for MTIC in the range of about 481 to about 2639 (ng.hr)/mL. In one embodiment, the arithmetic mean AUC (from time zero to infinity) for temozolomide is about 25 (μg.hr)/mL and the arithmetic mean AUC (from time zero to infinity) for MTIC is about 1004 (ng.hr)/mL.

In another preferred embodiment, the invention comprises a method for treating a patient having a proliferative disorder comprising the step of administering to the patient in need thereof a formulation comprising temozolomide or a pharmaceutically acceptable salt thereof by intravenous infusion over a period of about 1 hour to about 2 hours, wherein the administration of a single dose of 150 mg/m2 of the formulation achieves: (a) an arithmetic maximum plasma concentration (Cmax) of temozolomide in the range of about 5.5 to about 10.6 μg/mL and an arithmetic maximum plasma concentration (Cmax) of MTIC in the range of about 137 to about 916 ng/mL; and (b) wherein a plot of the plasma concentration of temozolomide versus time yields an arithmetic AUC (from time zero to infinity) for temozolomide in the range of about 17.6 to about 37.0 (μg.hr)/mL, and a plot of the plasma concentration of MTIC versus time yields an arithmetic AUC (from time zero to infinity) for MTIC in the range of about 481 to about 2639 (ng.hr)/mL. In one embodiment, the arithmetic mean maximum plasma concentration (Cmax) of temozolomide is about 7.4 μg/mL and the mean maximum plasma concentration (Cmax) of MTIC is about 320 ng/mL; and wherein the arithmetic mean AUC (from time zero to infinity) for temozolomide is about 25 (μg.hr)/mL and the arithmetic mean AUC (from time zero to infinity) for MTIC is about 1004 (ng.hr)/mL.

In a preferred embodiment, the proliferative disorder is glioma or melanoma, and the formulation is administered by intravenous infusion over a period of about 1.5 hours.

In some embodiment, the formulation comprising temozolomide or a pharmaceutically acceptable salt thereof is infused intravenously using a pump.

In some embodiments, the formulation comprising temozolomide or a pharmaceutically acceptable salt thereof further comprises: (a) at least one aqueous diluent; and (b) at least one dissolution enhancing agent sufficient to substantially dissolve the temozolomide, wherein the dissolution enhancing agent is selected from the group consisting urea, L-histidine, L-threonine, L-asparagine, L-serine and L-glutamine. In some embodiments, the formulation further comprises: The method of any one of claims 1-22, wherein the formulation further comprises mannitol, L-threonine, polysorbate-80, sodium citrate dehydrate and hydrochlorid acid.

In some embodiments, the formulation comprising temozolomide or a pharmaceutically acceptable salt thereof further comprises polysorbate 80, L-threonine and mannitol.

In some embodiments, the formulation comprising temozolomide or a pharmaceutically acceptable salt thereof is a lyophilized powder. In some embodiments, the lyophilized powder is reconstituted in sterile water. In some embodiments, the reconstituted formulation comprises 2.5 mg/mL of temozolomide.

In some embodiments, the formulation is administered at a dose of 75 mg/m2 per day for 42 consecutive days. In other embodiments, the formulation is administered at a dose of 150-200 mg/m2, per day for 5 consecutive days in a 28 day cycle. In yet other embodiments, the formulation is administered at a dose of 150-200 mg/m2 per day for 7 consecutive days in a 14 day cycle. (The recited doses are based on body surface area.)

In some embodiments, the patient receiving the formulation comprising temozolomide or a pharmaceutically acceptable salt thereof further receives concomitant focal radiotherapy. In some embodiments, the concomitant focal radiotherapy consists of 60 Gy administered in 30 fractions.

The invention also comprises a manufactured drug product for treating a proliferative disorder, which comprises: (a) a pharmaceutical formulation comprising temozolomide or a pharmaceutically acceptable salt thereof, wherein the formulation is in the form of a lyophilized power; and product information which comprises instructions for administering the pharmaceutical formulation by intravenous infusion over a period of about 1.5 hours (90 minutes). In one embodiment, the product information further comprises instructions for administering the formulation by intravenous infusion at a dose of 75 mg/m2 per day for 42 consecutive days. In one embodiment, the product information further comprises instructions for administering concomitant focal radiotherapy. In one embodiment, the concomitant focal radiotherapy consists of 60 Gy administered in 30 fractions. In another embodiment, the product information further comprises instructions for administering the formulation by intravenous infusion at dose of 150-200 mg/m2 per day for 5 consecutive days in a 28 day cycle. In yet another embodiment, the product information further comprises instructions for administering the formulation by intravenous infusion at dose of 150-200 mg/m2 per day for 7 consecutive days in a 14 day cycle.

In any of the above methods, the invention may further comprise the oral administration of a formulation comprising temozolomide.

BRIEF DESCRIPTION OF THE DRAWINGS

FIGURE 1 shows a population pharmacokinetic model was developed to characterize the disposition of both TMZ and MTIC following PO administration.

FIGURE 2 shows a plot of the plasma concentrations for TMZ (Fig. 2A) and MTIC (Fig. 2B) as predicted and observed following oral administration of TMZ. IPRE is the individual Prediction. CONC is Concentration. The line represents the unity line.

DETAILED DESCRIPTION OF THE INVENTION

In order that the invention herein described may be fully understood, the following detailed description is set forth.

Unless defined otherwise, all technical and scientific terms used herein have the same meaning as those commonly understood by one of ordinary skill in the art to which this invention belongs. Although methods and materials similar or equivalent to those described herein can be used in the practice or testing of the present invention, suitable methods and materials are described below. The materials, methods and examples are illustrative only, and are not intended to be limiting. All publications, patents and other documents mentioned herein are incorporated by reference in their entirety.

Throughout this specification, the word "comprise" or variations such as "comprises" or "comprising" will be understood to imply the inclusion of a stated integer or groups of integers but not the exclusion of any other integer or group of integers.

In order to further define the invention, the following terms and definitions are provided herein.

The term "pharmaceutically acceptable carrier of adjuvant" refers to a non-toxic carrier or adjuvant that may be administered to a patient, together with temolozimide, and that does not destroy the pharmacological activity thereof.

The term "treating" or "treatment" is intended to mean mitigating or alleviating the symptoms of a cell proliferative disorder in a mammal such as a human or the improvement of an ascertainable measurement associated with a cell proliferative disorder.

The term "patient" refers to an animal including a mammal (e.g., a human).

The term "proliferative disorder" may be a neoplasm. Such neoplasms are either benign or malignant.

The term "neoplasm" refers to a new, abnormal growth of cells or a growth of abnormal cells that reproduce faster than normal. A neoplasm creates an unstructured mass (a tumor) which can be either benign or malignant.

The term "benign" refers to a tumor that is noncancerous, *e.g.*, its cells do not invade surrounding tissues or metastasize to distant sites.

The term "malignant" refers to a tumor that is cancerous, metastastic, invades contiguous tissue or is no longer under normal cellular growth control.

The term "brain tumor" includes glioma, glioblastoma multiforme, ependymoma, astrocytoma, medulloblastoma, neuroglioma, oligodendroglioma and meningioma.

The term "bioavailability" refers to the rate and extent to which the active ingredient or active moiety is absorbed from a drug product and becomes available at the site of action.

The term "bioequivalence" refers to the absence of a significant difference in the rate and extent to which the active ingredient or active moiety in pharmaceutical equivalents or pharmaceutical alternatives becomes available at the site of drug action when administered at the same molar dose under similar conditions in an appropriately designed study.

The term "pharmacokinetics" refers to the process by which a drug is absorbed, distributed, metabolized, and eliminated by the body. Pharmacokinetic parameters include "maximum plasma concentration" or "Cmax," and "area under the plasma concentration time curve" or "AUC".

The term "blood plasma concentration" refers to the concentration of active ingredient in the plasma component of blood of the patient being studied.

The term "aqueous diluent(s)" refers to aqueous fluids suitable for injection into a patient.

The term "weight percentage" or "wt %" for purposes of this invention is calculated on a basis of total weight of the formulation.

The term "sample" refers to a specimen that is obtained as or isolated from tumor tissue, brain tissue, cerebrospinal fluid, blood, plasma, serum, lymph, lymph nodes, spleen, liver, bone marrow, or any other biological specimen containing either MGMT protein or nucleic acid of the MGMT gene.

The term "MGMT" refers to $O^6$-methylguanine-DNA methyltransferase. MGMT is also known as an $O^6$-alkyl-guanine-DNA-alkyltransferase (AGAT).

The term "GM-CSF" means a protein which (a) has an amino acid sequence that is substantially identical to the sequence of mature (i.e., lacking a signal peptide) human GM-CSF described by Lee et al., Proc. Natl. Acad. Sci. U.S.A., 82:4360 (1985) and (b) has biological activity that is common to native GM-CSF.

The term "substantial identity of amino acid sequences" means that the sequences are identical or differ by one or more amino acid alterations (deletions, additions, substitutions) that do not substantially impair biological activity.

Methods of Treating Proliferative Disorders

The present invention provides a method for treating a patient having a proliferative disorder by administering to the patient an intravenous formulation of temozolomide or a pharmaceutically acceptable salt thereof over a period of about 0.6 hours to about 2.9 hours.

Proliferative disorders include, but are not limited to, carcinoma, sarcoma, glioma, glioblastoma, brain cancer, brain tumors, melanoma, lung cancer, thyroid follicular cancer, pancreatic cancer, anaplastic astrocytoma, bladder cancer, myelodysplasia, prostate cancer, testicular cancer, lymphoma, leukemia, mycosis fungoides, head and neck cancer, breast cancer, ovarian cancer, colorectal and/or colon cancer, or esophageal cancer.

In some embodiments, the methods are used to treat a brain tumor, glioma, melanoma, lung cancer, lymphoma, colorectal and/or colon cancer, head and neck, breast cancer, ovarian cancer, or esophageal cancer. In some embodiments, the methods are used to treat a brain tumor. In some embodiments, the methods are used to treat glioma. In some aspects, the glioma is anaplastic astrocytoma or glioblastoma multiforme. In other embodiments, the methods are used to treat melanoma. In some embodiments, the methods are used to treat lung cancer. In some aspects, the lung cancer is non-small cell lung cancer. In some embodiments, the methods are used to treat lymphoma. In some embodiments, the methods are used to treat colorectal and/or colon cancer. In some embodiments, the methods are used to treat head and neck cancer. In some embodiments, the methods are used to treat breast cancer. In some embodiments, the methods are used to treat ovarian cancer. In some embodiments, the methods are used to treat esophageal cancer.

In some embodiments, the methods of the present invention are used to treat a patient having a proliferative disorder by administering an intravenous formulation over a period of about 0.6 hours to about 2.9 hours. In other embodiments, the intravenous formulation is infused over a period of about 0.8 hours to about 2.5 hours. In some embodiments, the intravenous formulation is infused over a period of about 0.9 hours to about 2 hours. In other embodiments, the intravenous formulation is infused over a period of about 1.25 hours to about 1.75 hours. In preferred embodiments, the intravenous formulation is infused over a period of about 1.35 hours to about 1.65 hours. In more preferred embodiments, the intravenous formulation is infused over a period of about 1.45 hours to about 1.55 hours. In yet more preferred embodiments, the intravenous formulation is administered over a period of about 1.5 hours. In some aspects of these embodiments, the proliferative disorder is selected from carcinoma, sarcoma, glioma, glioblastoma, brain cancer, brain tumors, melanoma, lung cancer, thyroid follicular cancer, pancreatic cancer, anaplastic astrocytoma, bladder cancer, myelodysplasia, prostate cancer, testicular cancer, lymphoma, leukemia, mycosis fungoides, head and neck cancer, breast cancer, ovarian cancer, colorectal and/or colon cancer, or esophageal cancer. In some aspects, the proliferative disorder is a brain tumor. In some aspects, the proliferative disorder is glioma. In some aspects, the proliferative disorder is anaplastic astrocytoma or glioblastoma multiforme. In other aspects, the proliferative disorder is melanoma. In some aspects, the proliferative disorder is lung cancer. In further aspects, the lung cancer is non-small cell lung cancer. In some aspects, the proliferative disorder is carcinoma. In some aspects, the proliferative disorder is sarcoma. In some aspects, the proliferative disorder is brain cancer. In some aspects, the proliferative disorder is thyroid follicular cancer. In some aspects, the proliferative disorder is pancreatic cancer. In some aspects, the proliferative disorder is bladder cancer. In some aspects, the proliferative disorder is myelodysplasia. In some aspects, the proliferative disorder is prostate cancer. In some aspects, the proliferative disorder is testicular cancer. In some aspects, the proliferative disorder is lymphoma. In some aspects, the proliferative disorder is leukemia. In some aspects, the proliferative disorder is mycosis fungoides. In some aspects, the proliferative disorder is head and neck cancer. In some aspects, the proliferative disorder is breast cancer. In some aspects, the proliferative disorder is ovarian cancer. In some aspects, the proliferative disorder is colorectal and/or colon cancer. In some aspects, the proliferative disorder is esophageal cancer.

Intravenous Formulations Used In The Methods

The intravenous formulations used in the methods of the present invention comprise temozolomide or a pharmaceutically acceptable salt thereof. Suitable pharmaceutically acceptable salts include, but are not limited to, those prepared from acids, such as hydrochloric acid, hydrobromic acid, phosphoric acid, sulfuric acid, maleic acid, citric acid, acetic acid, tartaric acid, succinic acid, oxalic acid, malic acid, glutamic acid, pamoic acid and the acid, as well as other acid related to pharmaceutically acceptable salts listed in Journal of Pharmaceutical Science, 66, 2 (1977).

In some embodiments, the temozolomide, or a pharmaceutically acceptable salt thereof, is present in the formulation in an amount ranging from about 1 wt % to about 50 wt %. In preferred embodiments, the temozolomide, or a pharmaceutically acceptable salt thereof, is present in the formulation in an amount ranging from about 2 wt % to about 30 wt %. In more preferred embodiments, the temozolomide, or a pharmaceutically acceptable salt thereof, is present in the formulation in an amount ranging from about 4 wt % to about 16 wt %.

In some embodiments, the intravenous formulations of the present invention further comprise at least one aqueous diluent and at least one dissolution enhancing agent sufficient to substantially dissolve the temozolomide or a pharmaceutically acceptable salt thereof.

According to this embodiment, the percentage of temozolomide, or a pharmaceutically acceptable salt thereof, dissolved in the pharmaceutical formulation can range from about 50% to about 100%. In preferred embodiments, the percentage ranges from about 75% to about 100%. In more preferred embodiments, the percentage is about 100%.

Suitable aqueous diluents include, but are not limited to, water, normal saline, 5% dextrose solution and mixtures thereof.

Suitable dissolution enhancing agents include, but are not limited to, urea, L-histidine, L-threonine, L-asparagine, L-serine, L-glutamine or mixtures thereof. [0001] The dissolution enhancing agent increases the rate in which the temozolomide, or a pharmaceutically acceptable salt thereof, dissolves in the aqueous diluent(s). The time to it takes to complete dissolution of temozolomide, or a pharmaceutically acceptable salt thereof, with a dissolution agent in at least one aqueous diluent in a 25 mg vial can range from about 30 seconds to about 90 seconds. In preferred embodiments, the dissolution time ranges from about 30 seconds to about 60 seconds. In more embodiments, the dissolution time is about 30 seconds.

In some embodiments, urea is used as the dissolution enhancing agent. According to this embodiment, the dissolution enhancing agent may be present in the formulation in an amount ranging from about 4 wt % to about 60 wt %. In preferred embodiments, the dissolution enhancing agent may be present in an amount ranging from about 8 wt % to about 30 wt %. In more preferred embodiments, the dissolution enhancing agent may be present in an amount ranging from about 12 wt % to about 22 wt %.

In some embodiments, the dissolution enhancing agent is L-histidine, L-threonine, L-asparagine, L-serine, L-glutamine or mixtures thereof. According to this embodiment, the dissolution enhancing agent may be present in an amount ranging from about 2 wt % to about 60 wt %. In preferred embodiments, the dissolution enhancing agent may be present in an amount ranging from about 4 wt % to about 40 wt %. In more preferred embodiments, the dissolution enhancing agent may be present in an amount ranging from about 8 wt % to about 20 wt %.

In some embodiments, the dissolution enhancing agent is L-histidine alone. According to this embodiment, the dissolution enhancing agent may be present in an amount ranging from about 1 wt % to about 30 wt %. In preferred embodiments, the dissolution enhancing agent may be present in an amount ranging from about 2 wt % to about 20 wt %. In more preferred embodiments, the dissolution enhancing agent may be present in an amount ranging from about 4 wt % to about 10 wt %.

In some embodiments, the intravenous formulation of the present invention further comprises at least one excipient. The excipient may used to increase the solubility of the temozolomide. Suitable excipients include, but are not limited to, polysorbates, polyethylene glycols (PEG), propylene glycols, polysorbates or suitable mixtures thereof.

In some embodiments, the excipient is a polysorbate. Suitable polysorbates include, but are not limited to, polysorbates having an average molecular weight ranging from about 500 g/mole to about 1900 g/mole. In preferred embodiments, the polysorbate has an average molecular weight ranging from about 800 g/mole to about 1600 g/mole. In more preferred embodiments, the polysorbate has an average molecular weight ranging from about 1000 g/mole to about 1400 g/mole. Examples of polysorbates include, but are not limited to, polysorbate 20, polysorbate 21, polysorbate 40, polysorbate 60, polysorbate 61, polysorbate 65, polysorbate 81, polysorbate 85, and polysorbate 120. In preferred embodiments, the polysorbate is polysorbate 20, polysorbate 80, or mixtures thereof.

In some embodiments, the excipient is PEG. Suitable PEGs include, but are not limited to, PEG having an average molecular weight ranging from about 200 g/mole to about 600 g/mole. In preferred embodiments, the PEG has an average molecular weight ranging from about 200 g/mole to about 500 g/mole. In more preferred embodiments, the PEG has an average molecular weight ranging from about 200 g/mole to about 400 g/mole. Examples of PEG, include but are not limited to, PEG 200, PEG 300, PEG 400, PEG 540, and PEG 600.

In some embodiments, the excipient is propylene glycol. Propylene glycol is a small molecule with a molecular weight of about 76.1 g/mole.

In some embodiments, the excipient is present in the intravenous formulation in an amount ranging from about 1 wt % to about 50 wt %. In preferred embodiments, the excipient is present in an amount ranging from about 2 wt % to about 30 wt %. In more preferred embodiments, the excipient is present in an amount ranging from about 4 wt % to about 16 wt %.

In some embodiments, the intravenous formulations of the present invention further comprise at least one bulking agent. Suitable bulking agents include, but are not limited to, mannitol, lactose, sucrose, sodium chloride, trehalose, dextrose, starch, hydroxyethylstarch (hetastarch), cellulose, cyclodextrins, glycine, and mixtures thereof. In some aspects of this embodiment, the bulking agent is mannitol.

In some embodiments, the bulking agent is present in the intravenous formulation in an amount ranging from about 20 wt % to about 80 wt %. In preferred embodiments, the bulking agent is present in an amount ranging from about 35 wt % to about 65 wt %. In more preferred embodiments, the bulking agent is present in an amount ranging from about 40 wt % to about 56 wt %.

In some embodiments, the intravenous formulations of the present invention further comprise at least one buffer. Suitable buffers include, but are not limited to, citrate buffers, lithium lactate, sodium lactate, potassium lactate, calcium lactate, lithium phosphate, sodium phosphate, potassium phosphate, calcium phosphate, lithium maleate, sodium maleate, potassium maleate, calcium maleate, lithium tartarate, sodium tartarate, potassium tartarate, calcium tartarate, lithium succinate, sodium succinate, potassium succinate, calcium succinate, lithium acetate, sodium acetate, potassium acetate, calcium acetate, and mixtures thereof.

In some embodiments, the buffer is a citrate buffer. Suitable citrate buffers include, but are not limited to, lithium citrate monohydrate, sodium citrate monohydrate, potassium citrate monohydrate, calcium citrate monohydrate, lithium citrate dihydrate, sodium citrate dihydrate, potassium citrate dihydrate, calcium citrate dihydrate, lithium citrate trihydrate, sodium citrate trihydrate, potassium citrate trihydrate, calcium citrate trihydrate, lithium citrate tetrahydrate, sodium citrate tetrahydrate, potassium citrate tetrahydrate, calcium citrate tetrahydrate, lithium citrate pentahydrate, sodium citrate pentahydrate, potassium citrate pentahydrate, calcium citrate pentahydrate, lithium citrate hexahydrate, sodium citrate hexahydrate, potassium citrate hexahydrate, calcium citrate hexahydrate, lithium citrate heptahydrate, sodium citrate heptahydrate, potassium citrate heptahydrate, and calcium citrate heptahydrate.

In some embodiments, the buffer is present in the intravenous formulation in an amount ranging from about 5 wt % to about 60 wt %. In preferred embodiments, the buffer is present in an amount ranging from about 10 wt % to about 40 wt %. In more preferred embodiments, the buffer is present in an amount ranging from about 15 wt % to about 28 wt %.

In some embodiments, the intravenous formulations of the present invention further comprise a pH adjuster. Suitable pH adjusters include, but are not limited to, hydrochloric acid, sodium hydroxide, citric acid, phosphoric acid, lactic acid, tartaric acid, succinic acid, and mixtures thereof. In some embodiments, the pH adjuster is hydrochloric acid.

In some embodiments, the pH adjuster is present in the intravenous formulation in an amount ranging from about 1 wt % to about 20 wt %. In preferred embodiments, the pH adjuster is present in an amount ranging from about 2 wt % to about 12 wt %. In more preferred embodiments, the pH adjuster is present in an amount ranging from about 4 wt % to about 8 wt %.

In some embodiments, the pH of the intravenous formulation of the present invention ranges from about 2.5 to about 6.0. In preferred embodiments, the pH ranges from about 3.0 to about 4.5. In more preferred embodiments, the pH ranges from about 3.8 to about 4.2.

In some embodiments, the intravenous formulation comprises temozolomide, an aqueous diluent, urea, hydrochloric acid, at least one citrate buffer, and mannitol.

In some embodiments, the intravenous formulation comprises temozolomide, urea, hydrochloric acid, a citrate buffer, mannitol and an aqueous diluent selected from water, normal saline, 5% dextrose solution and mixtures thereof. In some aspects of this embodiment, the temozolomide is present in an amount ranging from about 1 wt % to about 50 wt %, the hydrochloric acid is present in an amount ranging from about 1 wt % to about 20 wt %, the citrate buffer(s) is present in an amount ranging from about 5 wt % to about 60 wt %, the urea is present in an amount ranging from about 4 wt % to about 60 wt %, and the mannitol is present in an amount ranging from about 10 wt % to about 85 wt %.

In some embodiments, the intravenous formulation comprises temozolomide, polysorbate, hydrochloric acid, at least one citrate buffer, mannitol, water and a dissolution enhancing agent selected from the group consisting of L-histidine, L-threonine, L-asparagine, L-serine, and L-glutamine. In some aspects of this embodiment, the temozolomide is present in an amount ranging from about 1 wt % to about 50 wt %, the hydrochloric acid is present in an amount ranging from about 1 wt % to about 20 wt %, the citrate buffer(s) is present in an amount ranging from about 5 wt % to about 60 wt %, the polysorbate is present in an amount ranging from about 1 wt % to about 50 wt %, the dissolution enhancing agent is present in an amount ranging from about 2 wt % to about 60 wt %, and the mannitol is present in an amount ranging from about 15 wt % to about 85 wt %.

In some embodiments, the intravenous formulation comprises temozolomide, polysorbate, hydrochloric acid, at least one citrate buffer, mannitol, water and L-threonine. In some aspects of this embodiment, the intravenous formulation comprises temozolomide, polysorbate 80, hydrochloric acid, sodium citrate buffer, mannitol, water and L-threonine.

The intravenous formulations of the present invention may be prepared according to the methods described in U.S. patent 6,987,108, the entire content of which is incorporated by reference.

Dosing Regimens

The intravenous formulations of the present invention are administered to a patient according to a dosing regimen. It should be understood that the specific dosing regimen for any particular patient will depend on a variety of factors, including species, age, body weight, body surface area, height, general health, sex, diet, time of administration, rate of excretion, drug combination, specific disease being treated, the severity of the condition, the renal and hepatic function of the patient, the particular active ingredient or salt thereof employed, and the judgment of the treating physician.

In some embodiments, the intravenous formulations of the present invention are administered to treat a proliferative disorder according to one of the dosing regimens presented in Table 1. In some embodiments, the dosing regimen is 150-200 mg/m$^2$ per day for 5 days in a 28 day cycle. In other embodiments, the dosing regimen is 100 mg/m$^2$ per day for 14 days in a 21 day cycle. In other embodiments, the dosing regimen is 150 mg/m$^2$ per day for 7 days in a 14 day cycle. In yet other embodiments, the dosing regimen is 75 mg/m$^2$ per 42 days in a 56 day cycle.

In some aspects of these embodiments, the proliferative disorder is selected from carcinoma, sarcoma, glioma, glioblastoma, brain cancer, brain tumors, melanoma, lung cancer, thyroid follicular cancer, pancreatic cancer, anaplastic astrocytoma, bladder cancer, myelodysplasia, prostate cancer, testicular cancer, lymphoma, leukemia, mycosis fungoides, head and neck cancer, breast cancer, ovarian cancer, colorectal and/or colon cancer, or esophageal cancer. In some aspects, the proliferative disorder is a brain tumor. In some aspects, the proliferative disorder is glioma. In some aspects, the proliferative disorder is anaplastic astrocytoma or glioblastoma multiforme. In other aspects, the proliferative disorder is melanoma. In some aspects, the proliferative disorder is lung cancer. In further aspects, the lung cancer is non-small cell lung cancer. In some aspects, the proliferative disorder is carcinoma. In some aspects, the proliferative disorder is sarcoma. In some aspects, the proliferative disorder is brain cancer. In some aspects, the proliferative disorder is thyroid follicular cancer. In some aspects, the proliferative disorder is pancreatic cancer. In some aspects, the proliferative disorder is bladder cancer. In some aspects, the proliferative disorder is myelodysplasia. In some aspects, the proliferative disorder is prostate cancer. In some aspects, the proliferative disorder is testicular cancer. In some aspects, the proliferative disorder is lymphoma. In some aspects, the proliferative disorder is leukemia. In some aspects, the proliferative disorder is mycosis fungoides. In some aspects, the proliferative disorder is head and neck cancer. In some aspects, the proliferative disorder is breast cancer. In some aspects, the proliferative disorder is ovarian cancer. In some aspects, the proliferative disorder is colorectal and/or colon cancer. In some aspects, the proliferative disorder is esophageal cancer.

Table 1.

| Regimen No. | Dosing Regimen | Dosing Schedule | Total Dose (mg/m$^2$/4 wks) | Dose/wk (mg/m$^2$) | Dose Intensity |
|---|---|---|---|---|---|
| 1 | 5/28 | 150-200 mg/m$^2$, 5 days/28 day cycle (200 mg) | 1000 | 250 | 1 |
| 2 | High doses 250 mg/m$^2$ for 5/28 | 250 mg/m$^2$, 5/28, concomitant w/a growth factor | 1250 | 312 | 1.2 |
| 3 | 14/28 | 100 mg/m$^2$, 14 days/28 day 1400 cycle | 1400 | 350 | 1.4 |
| 4 | High doses 300 mg/m$^2$ for 5/28 | 300 mg/m$^2$, 5/28, concomitant w/a growth factor | 1500 | 375 | 1.5 |
| 5 | 21/28 | 75 mg/m$^2$, 21 days/28 day cycle | 1575 | 393.75 | 1.6 |
| 6 | 42/56 | 75 mg/m$^2$, 6 wks/ 8 wk cycle | 3150 | 393.75 | 1.6 |
| 7 | 21/28 | 85 mg/m$^2$, 21 days/28 day cycle | 1785 | 446.25 | 1.8 |
| 8 | High doses 350 mg/m$^2$ for 5/28 | 350 mg/m$^2$, 5/28, concomitant w/a growth factor | 1750 | 437.5 | 1.8 |
| 9 | 14 on/7 off | 100 mg/m$^2$, 14 days/21 day cycle | 1400* | 467 | 1.9 |
| 10 | High doses 400 mg/m$^2$ for 5/28 | 400 mg/m$^2$, 5/28, concomitant w/a growth factor | 2000 | 500 | 2.0 |

**TMZ Dosing Regimens and Dose Intensity**

(continued)

| TMZ Dosing Regimens and Dose Intensity | | | | | |
|---|---|---|---|---|---|
| Regimen No. | Dosing Regimen | Dosing Schedule | Total Dose $(mg/m^2/4\ wks)$ | Dose/wk $(mg/m^2)$ | Dose Intensity |
| 11 | 7/7 | 150 mg/m$^2$, 7 days/14 day cycle | 2100 | 525 | 2.1 |
| 12 | 21/28 | 100 mg/m$^2$, 21 days/28 day cycle | 2100 | 525 | 2.1 |
| 13 | 14/28 | 150 mg/m$^2$, 14 days/28 day cycle | 2100 | 525 | 2.1 |
| 14 | Continuous dosing | 75 mg/m$^2$, daily | 2100 | 525 | 2.1 |
| 15 | High doses 450 mg/m$^2$ for 5/28 | 450 mg/m$^2$, 5/28, concomitant w/a growth factor | 2250 | 562.5 | 2.25 |
| 16 | 14 on/7 off | 150 mg/m$^2$, 14 days/21 day cycle | 2100* | 700 | 2.8 |
| 17 | Continuous dosing | 100 mg/m$^2$, daily | 2800 | 700 | 2.8 |
| 18 | High doses 250 mg/m$^2$ for 7/7 | 250 mg/m$^2$, 7/7, concomitant w/a growth factor | 3500 | 875 | 3.5 |
| 19 | High doses 300 mg/m$^2$ for 7/7 | 300 mg/m$^2$, 7/7, concomitant w/a growth factor | 4200 | 1050 | 4.2 |
| *Represents total dose received in 3 week cycle | | | | | |

In some embodiments, the methods of the present invention are used to treat glioma. In some embodiments, the intravenous formulations are administered to a patient having glioma according to one of the dosing regimens presented in Table 1. In some embodiments, the dosing regimen is 150-200 mg/m$^2$ per day for 5 days in a 28 day cycle. In other embodiments, the dosing regimen is 100 mg/m$^2$ per day for 14 days in a 21 day cycle. In other embodiments, the dosing regimen is 150 mg/m$^2$ per day for 7 days in a 14 day cycle. In yet other embodiments, the dosing regimen is 75 mg/m$^2$ per 42 days in a 56 day cycle.

In some embodiments, the methods of the present invention are used to treat melanoma. In some embodiments, the intravenous formulations are administered to a patient having melanoma according to one of the dosing regimens presented in Table 1. In some embodiments, the dosing regimen is 150-200 mg/m$^2$ per day for 5 days in a 28 day cycle. In other embodiments, the dosing regimen is 100 mg/m$^2$ per day for 14 days in a 21 day cycle. In other embodiments, the dosing regimen is 150 mg/m$^2$ per day for 7 days in a 14 day cycle. In yet other embodiments, the dosing regimen is 75 mg/m$^2$ per 42 days in a 56 day cycle.

In some embodiments, the methods of the present invention are used to treat lung cancer. In some aspects of this embodiment, the lung cancer is non-small cell lung cancer. In some embodiments, the intravenous formulations are administered to a patient having lung cancer according to one of the dosing regimens presented in Table 1. In some embodiments, the dosing regimen is 150-200 mg/m$^2$ per day for 5 days in a 28 day cycle. In other embodiments, the dosing regimen is 100 mg/m$^2$ per day for 14 days in a 21 day cycle. In other embodiments, the dosing regimen is 150 mg/m$^2$ per day for 7 days in a 14 day cycle. In yet other embodiments, the dosing regimen is 75 mg/m$^2$ per 42 days in a 56 day cycle.

In some embodiments, the methods of the present invention are used to treat lymphoma. In some embodiments, the intravenous formulations are administered to a patient having lymphoma according to one of the dosing regimens presented in Table 1. In some embodiments, the dosing regimen is 150-200 mg/m$^2$ per day for 5 days in a 28 day cycle. In other embodiments, the dosing regimen is 100 mg/m$^2$ per day for 14 days in a 21 day cycle. In other embodiments, the dosing regimen is 150 mg/m$^2$ per day for 7 days in a 14 day cycle. In yet other embodiments, the dosing regimen is 75 mg/m$^2$ per 42 days in a 56 day cycle.

In some embodiments, the methods of the present invention are used to treat head and neck cancer. In some embodiments, the intravenous formulations are administered to a patient having head and neck cancer according to one of the dosing regimens presented in Table 1. In some embodiments, the dosing regimen is 150-200 mg/m$^2$ per day for 5 days in a 28 day cycle. In other embodiments, the dosing regimen is 100 mg/m$^2$ per day for 14 days in a 21 day cycle. In other embodiments, the dosing regimen is 150 mg/m$^2$ per day for 7 days in a 14 day cycle. In yet other embodiments, the dosing regimen is 75 mg/m$^2$ per 42 days in a 56 day cycle.

In some embodiments, the methods of the present invention are used to treat ovarian cancer. In some embodiments, the intravenous formulations are administered to a patient having ovarian cancer according to one of the dosing regimens presented in Table 1. In some embodiments, the dosing regimen is 150-200 mg/m$^2$ per day for 5 days in a 28 day cycle. In other embodiments, the dosing regimen is 100 mg/m$^2$ per day for 14 days in a 21 day cycle. In other embodiments, the dosing regimen is 150 mg/m$^2$ per day for 7 days in a 14 day cycle. In yet other embodiments, the dosing regimen is 75 mg/m$^2$ per 42 days in a 56 day cycle.

In some embodiments, the methods of the present invention are used to treat colorectal and/or colon cancer. In some embodiments, the intravenous formulations are administered to a patient having colorectal and/or colon cancer according to one of the dosing regimens presented in Table 1. In some embodiments, the dosing regimen is 150-200 mg/m$^2$ per day for 5 days in a 28 day cycle. In other embodiments, the dosing regimen is 100 mg/m$^2$ per day for 14 days in a 21 day cycle. In other embodiments, the dosing regimen is 150 mg/m$^2$ per day for 7 days in a 14 day cycle. In yet other embodiments, the dosing regimen is 75 mg/m$^2$ per 42 days in a 56 day cycle.

In some embodiments, the methods of the present invention are used to treat esophageal cancer. In some embodiments, the intravenous formulations are administered to a patient having esophageal cancer according to one of the dosing regimens presented in Table 1. In some embodiments, the dosing regimen is 150-200 mg/m$^2$ per day for 5 days in a 28 day cycle. In other embodiments, the dosing regimen is 100 mg/m$^2$ per day for 14 days in a 21 day cycle. In other embodiments, the dosing regimen is 150 mg/m$^2$ per day for 7 days in a 14 day cycle. In yet other embodiments, the dosing regimen is 75 mg/m$^2$ per 42 days in a 56 day cycle.

Methods For Administering One Or More Additional Therapeutic Agents

In some embodiments, the methods of the present invention comprise administering an intravenous formulation of temozolomide and one or more additional therapeutic agents. The one or more additional therapeutic agents may be administered by any route. In some embodiments, the one or more additional therapeutic agents are administered intravenously. In some aspects of this embodiment, the one or more additional therapeutic agents are administered by an intravenous injection. In other aspects, the one or more additional therapeutic agents are administered by an intravenous infusion.

Suitable additional therapeutic agents include, but are not limited to, growth factors, poly(ADP-ribose) polymerase(s) (PARP) inhibitors, O$^6$-benzylguanine (O$^6$BG), anti-emetic agents, steroids, farnesyl protein transferase inhibitors, P-glycoprotein (P-gp) inhibitors, cyclin-dependent kinase (CDK) inhibitors, checkpoint kinase-1 (CHK-1) inhibitors, other antineoplastic or anticancer agents and agents that alter the disposition of temozolomide to affect efficacy and/or safety.

In some embodiments, the intravenous formulations are administered in combination with a growth factor. Suitable growth factors include, but are not limited to, GM-CSF, G-CSF, IL-1, IL-3, IL-6, or erythropoietin. Non-limiting growth factors include Epogen® (epoetin alfa), Procrit®. (epoetin alfa), Neupogen® (filgrastim, a human G-CSF), Aranesp® (hyperglycosylated recombinant darbepoetin alfa), Neulasta® (also branded Neupopeg, pegylated recombinant filgrastim, pegfilgrastim), Albupoietin™ (a long-acting erythropoietin), and Albugranin™ (albumin G-CSF, a long-acting G-CSF). In some embodiments, the growth factor is G-CSF.

In some embodiments, the intravenous formulations are administered in combination with a poly(ADP-ribose) polymerase(s) (PARP) inhibitor. The PARP inhibitor may be administered either prior to, concomitantly with or after administration of the unit dosage forms of the present invention. Suitable PARP inhibitors include CEP-6800 (Cephalon; described in Miknyoczki et al., Mol Cancer Ther, 2(4):371-382 (2003)); 3-aminobenzamide (also known as 3-AB; Inotek; described in Liaudet et al., Br J Pharmacol, 133(8):1424-1430 (2001)); PJ34 (Inotek; described in Abdelkarim et al., Int J Mol Med, 7(3):255-260 (2001)); 5-iodo-6-amino-1,2-benzopyrone (also known as INH(2)BP; Inotek; described in Mabley et al., Br J Pharmacol, 133(6):909-919 (2001), GPI 15427 (described in Tentori et al., Int J Oncol, 26(2):415-422 (2005)); 1,5-dihydroxyisoquinoline (also known as DIQ; described in Walisser and Thies, Exp Cell Res, 251(2):401-413 (1999); 5-aminoisoquinolinone (also known as 5-AIQ; described in Di Paola et al., Eur J Pharmacol, 492(2-3):203-210 (2004); AG14361 (described in Bryant and Helleday, Biochem Soc Trans, 32(Pt 6):959-961 (2004); Veuger et al., Cancer Res, 63(18):6008-6015 (2003); and Veuger et al., Oncogene, 23(44):7322-7329 (2004)); ABT-472 (Abbott); INO-1001 (Inotek); AAI-028 (Novartis); KU-59436 (KuDOS; described in Farmer et al., "Targeting the DNA repair defect in BRCA mutant cells as a therapeutic strategy," Nature, 434(7035):917-921 (2005)); and those described in Jagtap et al., Crit Care Med, 30(5):1071-1082 (2002); Loh et al., Bioorg Med Chem Lett, 15(9):2235-2238 (2005); Ferraris et al., J Med Chem, 46 (14):3138-3151 (2003); Ferraris et al., Bioorg Med Chem Lett, 13(15):2513-2518 (2003); Ferraris et al., Bioorg Med Chem, 11(17):3695-3707 (2003); Li and Zhang IDrugs, 4(7):804-812 (2001); Steinhagen et al., Bioorg Med Chem Lett, 12(21):3187-3190 (2002)); WO 02/06284 (Novartis); and WO 02/06247 (Bayer).

In some embodiments, the intravenous formulations are administered in combination with $O^6$-benzylguanine ($O^6$BG) to accentuate hematopoietic toxicity. $O^6$BG can be administered either prior to, concomitantly with or after administration of the intravenous formulations of the present invention.

In some embodiments, the intravenous formulations are administered in combination with an anti-emetic agent. Suitable anti-emetic agents include, but are not limited to, Palonosetron, Tropisetron, Ondansetron, Granisetron, Bemesetron or a mixture thereof. In some embodiments, the amount of active anti-emetic substance in one dosage unit amounts to 2 to 10 mg, an amount of 5 to 8 mg active substance in one dosage unit being especially preferred. A daily dosage comprises generally an amount of active substance of 2 to 20 mg, particularly preferred is an amount of active substance of 5 to 16 mg. In some embodiments, a neurokinin-1 antagonist (NK-1 antagonist) such as aprepitant may be administered either alone or in combination with a steroid such as dexamethasone in conjunction with an anti-emetic agent. In other embodiments, the intravenous formulations are administered in combination with an NK-1 antagonist alone or with a steroid.

In some embodiments, the intravenous formulations are administered in combination with a farnesyl protein transferase inhibitor.

In some embodiments, the intravenous formulations are administered in combination with a P-gp inhibitor. Suitable P-gp inhibitors include Actinomycin D, Amiodarone, Atorvastatin, Bromocriptine, Carvedilol, Clarithromycin, Chloropromazine, Colchines, Cyclosporine, Desmethylsertraline, Dipyridamole, Doxorubicin, Emetine, Erythromycin, Esomeprazole, Fenofibrate, Flupenthixol, Fluoxetine, Indinavir, Itraconazole, Ketoconazole, Lansoprazole, Mefloquine, Megestrol acetate, Methadone, 10,11-methanodibenzosuberane, Nelfinavir, Nicardipine, Omeprazole, Pantoprazole, Paroxetine, Pentazocine, phenothiazines, Progesterone, Propafenone, Quinidine, Reserpine, Ritonavir, Saquinavir, Sertaline, Sirolimus, Spironolactone, Tacrolimus, Tamoxifen, Valspodar, Verapamil, Vinblastine, Vitamin E-TGPS, acridine derivatives such as GF120918, FK506, VX 710, LY335979, PSC-833, GF-102,918 and other steroids, and P-gp inhibitors disclosed in United States patent 6,703,400, the content of which is incorporated by reference in its entirety.

In some embodiments, the intravenous formulations are administered in combination with a CDK inhibitor. Suitable CDK inhibitors include flavopiridol, olomoucine, roscovitine, CDK inhibitors disclosed in United States patent 7,119,200, United States patent 7,161,003, United States patent 7,166,602, United States patent 7,196,078, United States patent. 6,107,305, K. S. Kim et al, J. Med. Chem. 45 (2002) 3905 3927, WO 02/10162, WO016465 and WO016465 all of which are incorporated by reference in their entirety.

In some embodiments, the intravenous formulations are administered in combination with a CHK-1 inhibitor. Suitable CHK-1 inhibitors include XL-844, CDK-1 inhibitors disclosed in Sanchez, Y. et. al. (1997) Science 277: 1497 1501 and Flaggs, G. et. al. (1997) Current Biology 7:977 986; U.S. Pat. Nos. 6,413,755, 6,383,744, and 6,211,164; and International Publication Nos. WO 01/16306, WO 01/21771, WO 00/16781, and WO 02/070494), all of which are incorporated by reference in their entirety.

Other CDK and CHK inhibitors useful in the present invention are described in WO2007/044449; WO2007/044441; WO2007/044426; WO2007/044420; WO2007/044410; WO2007/044407; WO2007/044401; and W02007/041712, all of which are incorporated by reference in their entirety.

In other embodiments, the intravenous formulations are administered with another antineoplastic agent. Suitable antineoplastic agents include, but are not limited to, Uracil Mustard, Chlormethine, Cyclophosphamide, Ifosfamide, Melphalan, Chlorambucil, Pipobroman, Triethylenemelamine, Triethylenethiophosphoramine, Busulfan, Carmustine, Lomustine, Streptozocin, Dacarbazine, Methotrexate, 5-Fluorouracil, Floxuridine, Cytarabine, 6-Mercaptopurine, 6-Thioguanine, Fludarabine phosphate, Pentostatine, Gemcitabine, Vinblastine, Vincristine, Vindesine, Bleomycin, Dactinomycin, Daunorubicin, Doxorubicin, Epirubicin, Idarubicin, Paclitaxel, Mithramycin, Deoxycoformycin, Mitomycin-C, L-Asparaginase, Interferons, Etoposide, Teniposide 17α-Ethinylestradiol, Diethylstilbestrol, Testosterone, Prednisone, Fluoxymesterone, Dromostanolone propionate, Testolactone, Megestrolacetate, Tamoxifen, Methylprednisolone, Methyltestosterone, Prednisolone, Triamcinolone, Chlorotrianisene, Hydroxyprogesterone, Aminoglutethimide, Estramustine, Medroxyprogesteroneacetate, Leuprolide, Flutamide, Toremifene, Goserelin, Cisplatin, Carboplatin, Hydroxyurea, Amsacrine, Procarbazine, Mitotane, Mitoxantrone, Levamisole, Navelbene, Anastrazole, Letrazole, Capecitabine, Reloxafine, Droloxafine, Hexamethylmelamine, Oxaliplatin (Eloxatin®), Iressa (gefinitib, Zd1839), XELODA® (capecitabine), Tarceva® (erlotinib), Azacitidine (5-Azacytidine; 5-AzaC), and mixtures thereof.

In some embodiments, the intravenous formulations may be administered with other anti-cancer agents such as the ones disclosed in United States Patents 5,824,346, 5,939,098, 5,942,247, 6,096,757, 6,251,886, 6,316,462, 6,333,333, 6,346,524, and 6,703,400, all of which are incorporated by reference in their entirety.

Methods For Assessing Methylation State Of MGMT Gene

Temozolomide, as an alkylating agent, causes cell death by binding to DNA which structurally distorts the DNA helical structure preventing DNA transcription and translation. In normal cells, the damaging action of alkylating agents can be repaired by cellular DNA repair enzymes, in particular MGMT. The level of MGMT varies in tumor cells, even among tumors of the same type. The gene encoding MGMT is not commonly mutated or deleted. Rather, low levels of MGMT in tumor cells are due to an epigenetic modification; the MGMT promoter region is methylated, thus inhibiting transcription of the MGMT gene and preventing expression of MGMT.

U.S. Publication 20060100188, the entire content of which is incorporated by reference, discloses methods for treating cancer in a patient comprising administering temozolomide according to improved dosing regimens and/or schedules based on the patient's MGMT level.

In some embodiments, the dosing regimen is based upon the methylation state of the MGMT gene in a sample obtained from the patient. In some embodiments, the methylation state is assessed by a determination of whether the MGMT gene is methylated. In other embodiments, the methylation state is assessed by a quantitative determination of the level of methylation of the MGMT gene. In other embodiments, the methylation state is assessed by determination of whether MGMT protein is expressed. In yet other embodiments, the methylation states is assessed by a determination of the level of MGMT protein expressed or measurement of the enzymatic activity of MGMT in the patient sample.

Assessing whether the MGMT gene is methylated may be performed using any method known to one skilled in the art. Techniques useful for detecting methylation of a gene or nucleic acid include, but are not limited to, those described by Ahrendt et al., J. Natl. Cancer Inst., 91:332-339 (1999); Belsinky et al., Proc. Natl. Acad. Sci. U.S.A., 95: 11891-11896 (1998), Clark et al., Nucleic Acids Res., 22:2990-2997 (1994); Herman et al., Proc Natl Acad Sci U.S.A., 93:9821-9826 (1996); Xiong and Laird, Nucleic Acids Res., 25:2532-2534 (1997); Eads et al., Nuc. Acids. Res., 28:e32 (2002); Cottrell et al., Nucleic Acids Res., 32:1-8 (2004).

Methylation-specific PCR (MSP) can rapidly assess the methylation status of virtually any group of CpG sites within a CpG island, independent of the use of methylation-sensitive restriction enzymes. See, MSP; Herman et al., Proc. Natl. Acad Sci USA, 93(18):9821-9826 (1996); Esteller et al., Cancer Res., 59:793-797 (1999)) see also U.S. Pat. No. 5,786,146, issued Jul. 28, 1998; U.S. Pat. No. 6,017,704, issued Jan. 25, 2000; U.S. Pat. No. 6,200,756, issued Mar. 13, 2001; and U.S. Pat. No 6,265,171, issued Jul. 24, 2001; U.S. Pat. No. 6,773,897 issued Aug. 10, 2004; the entire contents of each of which is incorporated herein by reference. The MSP assay entails initial modification of DNA by sodium bisulfite, converting all unmethylated, but not methylated, cytosines to uracil, and subsequent amplification with primers specific for methylated versus unmethylated DNA. MSP requires only small quantities of DNA, is sensitive to 0.1 % methylated alleles of a given CpG island locus, and may be performed on DNA extracted from paraffin-embedded samples. MSP eliminates the false positive results inherent to previous PCR-based approaches that relied on differential restriction enzyme cleavage to distinguish methylated from unmethylated DNA. This method is very simple and can be used on small amounts of tissue or a few cells. As would be understood by those skilled in the art, if the gene encoding MGMT is not methylated, the MGMT protein is expressed and can be detected (e.g., by Western blot, immuno-histochemical techniques or enzymatic assays for MGMT activity, etc.) as detailed below herein.

An illustrative example of a Western blot assay useful for this embodiment of the invention to measure the level of MGMT protein in patient samples is presented in U.S. Pat. No. 5,817,514 by Li et al., the entire disclosure of which is incorporated herein by reference. Li et al. described monoclonal antibodies able to specifically bind either to native human MGMT protein or to human MGMT protein having an active site which is alkylated.

An illustrative example of an immunohistochemical technique useful for this embodiment of the invention to measure the level of MGMT protein in patient samples is presented in U.S. Pat. No. 5,407,804, the entire disclosure of which is incorporated herein by reference. Monoclonal antibodies are disclosed which are able to specifically bind to the MGMT protein in single cell preparations (immunohistochemical staining assays) and in cell-extracts (immunoassays). The use of fluorescent read out coupled with digitization of the cell image is described and allows for quantitative measurement of MGMT levels in patient and control samples, including but not limited to tumor biopsy samples.

Useful techniques for measuring the enzymatic activity of MGMT protein include but are not limited to methods described by: Myrnes et al., Carcinogenesis, 5:1061-1064 (1984); Futscher et al., Cancer Comm., 1: 65-73 (1989); Kreklaw et al., J. Pharmacol. Exper. Ther., 297(2):524-530 (2001); and Nagel et al., Anal. Biochem., 321(1):38-43 (2003), the entire disclosures of which are incorporated herein in their entireties.

The level of MGMT protein expressed in a sample obtained from a patient may be assessed by measuring MGMT protein, e.g., by Western blot using an antibody specific to MGMT, see for example, U.S. Pat. No. 5,817,514. The level of MGMT expressed in a sample may also be assessed by measuring the MGMT protein using an immunohistochemistry technique on a defined number of patient cells, e.g., employing a labeled antibody specific for MGMT and comparing the level with that expressed by the same defined number of normal lymphocytes known to express MGMT (see, for example, U.S. Pat. No. 5,407,804 by Yarosh for a description of useful quantitative immunohistochemical assays). Alternatively, the level of MGMT may be assessed by enzymatic assay, *i.e.*, the ability to methylate the $O^6$ or $N^7$ guanine position of DNA. In each of these methods, the measured level of MGMT protein expressed is compared to that expressed by normal lymphocytes known to express MGMT. Patient MGMT protein levels are classified as follows: Low=0-30% of the MGMT expressed by normal lymphocytes; Moderate=31-70% of the MGMT expressed by normal lymphocytes; and High=71-300% or higher of the MGMT expressed by normal lymphocytes. Patient MGMT levels are classified as follows: Low=0-30% of the MGMT enzymatic activity of normal lymphocytes; Moderate=31-70% of the MGMT enzymatic activity of normal lymphocytes; and High=71-300% or higher of the MGMT enzymatic activity of normal lymphocytes.

The specific activity of MGMT may be assessed and based on a comparison with cell lines known to express MGMT classified as follows: Low=less than 20 fmol/mg; Moderate=20-60 fmol/mg; or High=greater than 60 fmol/mg; where the specific activity of MGMT in LOX cells is 6-9 fmol/mg, in DAOY cells is 60-100 fmol/mg, and in A375 cells is 80-150 fmol/mg.

The level of methylation of MGMT may be assessed by quantitative determination of the methylation of the gene encoding MGMT. The quantitative technique called COBRA (Xiong et al., Nuc. Acids Res., 25:2532-2534 (1997)) may be used in this determination. The "methyl light" technique of Eads et al., Nuc. Acids Res., 28(8):e32 (2000); U.S. Pat. No. 6,331,393 may also be used. The level of methylation of gene encoding MGMT in cells of the patient is compared to that of an equivalent number of cells of normal lymphocytes known to express MGMT. As would be understood by those skilled in the art, normal lymphocytes expressing MGMT have a low level of methylation of the MGMT gene; conversely, cells with high levels of methylation of the MGMT gene express low levels of the MGMT protein (see for example, Costello et al., J. Biol. Chem., 269(25):17228-17237 (1994); Qian et al., Carcinogen, 16(6):1385-1390 (1995)). Patient methylated MGMT gene levels are classified as follows: Low=0-20% of the CpGs in the promoter region of the MGMT gene are methylated; Moderate=21-50% of the CpGs in the promoter region of the MGMT gene are methylated; and High=51-100% of the CpGs in the promoter region of the MGMT gene are methylated.

COBRA may be used to determine quantitatively DNA methylation levels at specific gene loci in small amounts of genomic DNA. Restriction enzyme digestion is used to reveal methylation-dependent sequence differences in PCR products of sodium bisulfite-treated DNA. (Tano et al., Proc. Natl. Acad. Sci. USA, 87:686-690 (1990) describe isolation and sequence of the human MGMT gene). Methylation levels in original DNA sample are represented by the relative amounts of digested and undigested PCR product in a linearly quantitative fashion across a wide spectrum of DNA methylation levels. This technique may be reliably applied to DNA obtained from microdissected paraffin-embedded tissue samples. COBRA thus combines the powerful features of ease of use, quantitative accuracy, and compatibility with paraffin sections.

An illustrative example of a RT-PCR assay useful for assessing the level of MGMT mRNA is described in Watts et al., Mol. Cell. Biol., 17(9):5612-5619 (1997). In brief, total cellular RNA is isolated by guanidium isothiocyanate cell lysis followed by centrifugation through a 5.7 M CsCl gradient for 2.5 hr at 205,000xg. RNA is quantitated in a Beckman TL-100 spectrophotometer by measurements of absorbance at 260 nm. Total cellular RNA is reverse transcribed by incubating a 40 $\mu$l reaction mixture composed of 200 ng ofRNA; 1xPCR buffer (10 mM Tris [pH 8.3], 50 mM KCl, 1.5 mM MgCl$_2$); 1 mM each dATP, dCTP, dGTP, and dTTP; 200 pmol of random hexamer, 40 U of RNasin, and 24 U of avian myeloblastosis virus reverse transcriptase (Boehringer Mannheim, Indianapolis, Ind.) at 42° C for 60 min. The reaction is then stopped by incubation at 99° C for 10 min. MGMT-specific PCR is performed by adding 80 $\mu$l of ampli-fication reaction buffer (1x PCR buffer, 25 pmol of MGMT-specific primers and/or a control sequence, and 2 U of Taq DNA polymerase) to 20 $\mu$l of the reverse transcription reaction mixture followed by incubation at 94° C for 5 min; 30 cycles of 94° C. for 1 min, 60° C. for 15 s, and 72° C. for 1 min; a final extension at 72° C. for 5 min; and a quick chill to 4° C. For example, the upstream primer sequence from exon 4 (nt 665 to 684) of the MGMT gene can be used. Nucleotide positions can be derived from the cDNA sequence (Tano et al., Proc. Natl. Acad. Sci. USA, 87:686-690 (1990)). A control primer sequence can be employed in the same cDNA reaction (e.g., primers for the histone 3.3 gene). For analysis, 10% of the respective PCR products are separated through a 3% agarose gel and visualized by ethidium bromide staining.

Methods Of Treating A Patient With A Proliferative Disorder Based Upon <u>Methylation State Of MGMT Gene</u>

The present invention provides a method for treating a patient having a proliferative disorder using an intravenous formulation of temozolomide or a pharmaceutically acceptable salt thereof. In some embodiments, the intravenous formulations are used to treat patients having a proliferative disorder selected from carcinoma, sarcoma, glioma, brain cancer, brain tumors, melanoma, lung cancer, thyroid follicular cancer, pancreatic cancer, bladder cancer, myelodysplasia, prostate cancer, testicular cancer, lymphoma, leukemia, mycosis fungoides, head and neck cancer, breast cancer, ovarian cancer, colorectal and/or colon cancer, or esophageal cancer. In some embodiments, the proliferative disorder is a brain tumor. In some aspects, the brain tumor is glioma. In some aspects, the glioma is anaplastic astrocytoma or glioblastoma multiforme. In other embodiments, the proliferative disorder is melanoma. In some embodiments, the proliferative disorder is lung cancer. In some aspects, the lung cancer is non-small cell lung cancer. In some embodiments, the proliferative disorder is carcinoma. In some embodiments, the proliferative disorder is sarcoma. In some embodiments, the proliferative disorder is brain cancer. In some embodiments, the proliferative disorder is thyroid follicular cancer. In some embodiments, the proliferative disorder is pancreatic cancer. In some embodiments, the proliferative disorder is bladder cancer. In some embodiments, the proliferative disorder is myelodysplasia. In some embodiments, the proliferative disorder is prostate cancer. In some embodiments, the proliferative disorder is testicular cancer. In some embodiments, the proliferative disorder is lymphoma. In some embodiments, the proliferative disorder is leukemia. In some embodiments, the proliferative disorder is mycosis fungoides. In some embodiments, the proliferative disorder is head and neck cancer. In some embodiments, the proliferative disorder is breast cancer. In some embodiments, the proliferative disorder is ovarian cancer. In some embodiments, the proliferative disorder is colorectal and/or colon cancer. In some embodiments, the proliferative disorder is esophageal cancer.

The intravenous formulation is administered according to a dosing regimen. In some embodiments, the dosing regimen is based upon the detection of methylated MGMT gene in a sample. When methylation of the MGMT gene is detected in a sample obtained from a patient having a proliferative disorder, the dosing regimen is 150-200 mg/m$^2$ per day for 5 days in a 28 day cycle. When methylation is not detected, the dosing regimen is (i) 100 mg/m$^2$ per day for 14 days in a 21 day cycle; (ii) 150 mg/m$^2$ per day for 7 days in a 14 day cycle; or (iii) 100 mg/m$^2$ per day for 21 days in a 28 day cycle. In some embodiments, the sample is a tumor biopsy sample. In some embodiments, the MGMT gene is detected using MSP.

In some embodiments, the dosing regimen is based upon the degree or level of methylation of MGMT gene detected in a sample. When the degree or level of methylation is Low, the dosing regimen is (i) 85 mg/m$^2$ per 21 days in a 28 day cycle; (ii) 350 mg/m$^2$ per day for 5 days in a 28 day cycle in combination with a growth factor; (iii) 100 mg/m$^2$ per day for 14 days in a 21 day cycle; (iv) 400 mg/m$^2$ per day for 5 days in a 28 day cycle in combination with a growth factor; (v) 150 mg/m$^2$ per day for 7 days in a 14 day cycle; (vi) 100 mg/m$^2$ per day for 21 days in a 28 day cycle; (vii) 150 mg/m$^2$ per day for 14 days in a 28 day cycle; (viii) 75 mg/m$^2$ per day daily; (ix) 450 mg/m$^2$ per day for 5 days in a 28 day cycle in combination with a growth factor; (x) 150 mg/m$^2$ per day for 14 days in a 21 day cycle; (xi) 100 mg/m$^2$ per day daily; (xii) 250 mg/m$^2$ per day for 7 days in a 14 day cycle in combination with a growth factor; or (xiii) 300 mg/m$^2$ per day for 7 days in a 14 day cycle in combination with a growth factor. In some aspects, the dosing regimen is (i) 100 mg/m$^2$ per day for 14 days in a 21 day cycle; (ii) 400 mg/m$^2$ per day for 5 days in a 28 day cycle in combination with a growth factor; (iii) 150 mg/m$^2$ per day for 7 days in a 14 day cycle. When the degree or level of methylation is Moderate, the dosing regimen is (i) 100 mg/m$^2$ per day for 14 days in a 28 day cycle; (ii) 300 mg/m$^2$ per day for 5 days in a 28 day cycle in combination with a growth factor; (iii) 75 mg/m$^2$ per day for 21 days in a 28 day cycle; or (iv) 75 mg/m$^2$ per 42 days in a 56 day cycle. When the degree or level of methylation is High, the dosing regimen is (i) 150-200 mg/m$^2$ per day for 5 days in a 28 day cycle; or (ii) 250 mg/m$^2$ per day for 5 days in a 28 day cycle in combination with a growth factor. In some embodiments, the sample is a tumor biopsy sample. In some embodiments, the MGMT gene is detected using MSP.

In other embodiments, the dosing regimen is based upon the presence or absence of MGMT protein. When the MGMT protein is not detected in a sample obtained from a patient having a proliferative disorder, the dosing regimen is 150-200 mg/m$^2$ per day for 5 days in a 28 day cycle. When the MGMT protein is detected, the dosing regimen is selected from (i) 100 mg/m$^2$ per day for 14 days in a 21 day cycle; 150 mg/m$^2$ per day for 7 days in a 14 day cycle; or (iii) 100 mg/m$^2$ per day for 21 days in a 28 day cycle. In some embodiments, the sample is a tumor biopsy sample. In some embodiments, the MGMT protein is detected using Western blot immunoassay, an immunohistochemical technique, or an enzymatic assay for MGMT protein.

In yet other embodiments, the dosing regimen is based upon the level or activity of the MGMT protein detected in a sample. When the level or enzymatic activity of the MGMT protein detected in a sample obtained from the patient is Low, compared to that of normal lymphocytes, the dosing regimen is (i) 150-200 mg/m$^2$ per day for 5 days in a 28 day cycle; or (ii) 250 mg/m$^2$ per day for 5 days in a 28 day cycle in combination with a growth factor. When the level or enzymatic activity of the MGMT protein is Moderate, the dosing regimen is (i) 100 mg/m$^2$ per day for 14 days in a 28 day cycle; (ii) 300 mg/m$^2$ per day for 5 days in a 28 day cycle in combination with a growth factor; (iii) 75 mg/m$^2$ per day for 21 days in a 28 day cycle; or (iv) 75 mg/m$^2$ per 42 days in a 56 day cycle. When the level or enzymatic activity of the MGMT protein detected in a sample obtained from the patient is High, the dosing regimen is selected from (i) 100 mg/m$^2$ per day for 14 days in a 21 day cycle; (ii) 150 mg/m$^2$ per day for 7 days in a 14 day cycle; or (iii) 100 mg/m$^2$ per day for 21 days in a 28 day cycle. In some embodiments, the sample is a tumor biopsy sample.

Methods Of Treating A Patient With Glioma Based Upon Methylation State Of <u>MGMT Gene</u>

The present invention also provides a method of treating of a patient with glioma using an intravenous formulation of temozolomide or a pharmaceutically acceptable salt thereof. The intravenous formulation is administered according to a dosing regimen. In some embodiments, the dosing regimen is based upon the detection of methylated MGMT gene in a sample. When methylation of the MGMT gene is detected in a sample obtained from a patient having a glioma, the dosing regimen is 150-200 mg/m$^2$ per day for 5 days in a 28 day cycle. When methylation is not detected, the dosing regimen is (i) 100 mg/m$^2$ per day for 14 days in a 21 day cycle; (ii) 150 mg/m$^2$ per day for 7 days in a 14 day cycle; or (iii) 100 mg/m$^2$ per day for 21 days in a 28 day cycle. In some embodiments, the sample is a tumor biopsy sample. In some embodiments, the MGMT gene is detected using MSP.

In some embodiments, the dosing regimen is based upon the degree or level of methylation of MGMT gene detected in a sample. When the degree or level of methylation is Low, the dosing regimen is (i) 85 mg/m$^2$ per 21 days in a 28 day cycle; (ii) 350 mg/m$^2$ per day for 5 days in a 28 day cycle in combination with a growth factor; (iii) 100 mg/m$^2$ per day for 14 days in a 21 day cycle; (iv) 400 mg/m$^2$ per day for 5 days in a 28 day cycle in combination with a growth factor; (v) 150 mg/m$^2$ per day for 7 days in a 14 day cycle; (vi) 100 mg/m$^2$ per day for 21 days in a 28 day cycle; (vii) 150 mg/m$^2$ per day for 14 days in a 28 day cycle; (viii) 75 mg/m$^2$ per day daily; (ix) 450 mg/m$^2$ per day for 5 days in a 28 day cycle in combination with a growth factor; (x) 150 mg/m$^2$ per day for 14 days in a 21 day cycle; (xi) 100 mg/m$^2$ per day daily; (xii) 250 mg/m$^2$ per day for 7 days in a 14 day cycle in combination with a growth factor; or (xiii) 300 mg/m$^2$ per day for 7 days in a 14 day cycle in combination with a growth factor. In some aspects, the dosing regimen is (i) 100 mg/m$^2$ per day for 14 days in a 21 day cycle; (ii) 400 mg/m$^2$ per day for 5 days in a 28 day cycle in combination with a growth factor; (iii) 150 mg/m$^2$ per day for 7 days in a 14 day cycle. When the degree or level of methylation is Moderate, the dosing regimen is (i) 100 mg/m$^2$ per day for 14 days in a 28 day cycle; (ii) 300 mg/m$^2$ per day for 5 days in a 28 day cycle in combination with a growth factor; (iii) 75 mg/m$^2$ per day for 21 days in a 28 day cycle; or (iv) 75 mg/m$^2$ per 42 days in a 56 day cycle. When the degree or level of methylation is High, the dosing regimen is (i) 150-200 mg/m$^2$ per day for 5 days in a 28 day cycle; or (ii) 250 mg/m$^2$ per day for 5 days in a 28 day cycle in combination with a growth factor. In some embodiments, the sample is a tumor biopsy sample. In some embodiments, the MGMT gene is detected using MSP.

In other embodiments, the dosing regimen is based upon the presence or absence of MGMT protein. When the MGMT protein is not detected in a sample obtained from a patient having a glioma, the dosing regimen is 150-200 mg/m$^2$ per day for 5 days in a 28 day cycle. When the MGMT protein is detected, the dosing regimen is selected from (i) 100 mg/m$^2$ per day for 14 days in a 21 day cycle; 150 mg/m$^2$ per day for 7 days in a 14 day cycle; or (iii) 100 mg/m$^2$ per day for 21 days in a 28 day cycle. In some embodiments, the sample is a tumor biopsy sample. In some embodiments, the MGMT protein is detected using Western blot immunoassay, an immunohistochemical technique, or an enzymatic assay for MGMT protein.

In yet other embodiments, the dosing regimen is based upon the level or activity of the MGMT protein detected in a sample. When the level or enzymatic activity of the MGMT protein detected in a sample obtained from the patient is Low, compared to that of normal lymphocytes, the dosing regimen is (i) 150-200 mg/m$^2$ per day for 5 days in a 28 day cycle; or (ii) 250 mg/m$^2$ per day for 5 days in a 28 day cycle in combination with a growth factor. When the level or enzymatic activity of the MGMT protein is Moderate, the dosing regimen is (i) 100 mg/m$^2$ per day for 14 days in a 28 day cycle; (ii) 300 mg/m$^2$ per day for 5 days in a 28 day cycle in combination with a growth factor; (iii) 75 mg/m$^2$ per day for 21 days in a 28 day cycle; or (iv) 75 mg/m$^2$ per 42 days in a 56 day cycle. When the level or enzymatic activity of the MGMT protein detected in a sample obtained from the patient is High, the dosing regimen is selected from (i) 100 mg/m$^2$ per day for 14 days in a 21 day cycle; (ii) 150 mg/m$^2$ per day for 7 days in a 14 day cycle; or (iii) 100 mg/m$^2$ per day for 21 days in a 28 day cycle. In some embodiments, the sample is a tumor biopsy sample.

Methods Of Treating A Patient With Melanoma Based Upon Methylation State Of <u>MGMT Gene</u>

The present invention also provides a method of treating of a patient with melanoma using an intravenous formulation of temozolomide or a pharmaceutically acceptable salt thereof. The intravenous formulation is administered according to a dosing regimen. In some embodiments, the dosing regimen is based upon the detection of methylated MGMT gene in a sample. When methylation of the MGMT gene is detected in a sample obtained from a patient having melanoma, the dosing regimen is 150-200 mg/m$^2$ per day for 5 days in a 28 day cycle. When methylation is not detected, the dosing regimen is (i) 100 mg/m$^2$ per day for 14 days in a 21 day cycle; (ii) 150 mg/m$^2$ per day for 7 days in a 14 day cycle; or (iii) 100 mg/m$^2$ per day for 21 days in a 28 day cycle. In some embodiments, the sample is a tumor biopsy sample. In some embodiments, the MGMT gene is detected using MSP.

In some embodiments, the dosing regimen is based upon the degree or level of methylation of MGMT gene detected in a sample. When the degree or level of methylation is Low, the dosing regimen is (i) 85 mg/m$^2$ per 21 days in a 28 day cycle; (ii) 350 mg/m$^2$ per day for 5 days in a 28 day cycle in combination with a growth factor; (iii) 100 mg/m$^2$ per day for 14 days in a 21 day cycle; (iv) 400 mg/m$^2$ per day for 5 days in a 28 day cycle in combination with a growth factor; (v) 150 mg/m$^2$ per day for 7 days in a 14 day cycle; (vi) 100 mg/m$^2$ per day for 21 days in a 28 day cycle; (vii) 150 mg/m$^2$ per day for 14 days in a 28 day cycle; (viii) 75 mg/m$^2$ per day daily; (ix) 450 mg/m$^2$ per day for 5 days in a 28 day cycle in combination with a growth factor; (x) 150 mg/m$^2$ per day for 14 days in a 21 day cycle; (xi) 100 mg/m$^2$ per day daily; (xii) 250 mg/m$^2$ per day for 7 days in a 14 day cycle in combination with a growth factor; or (xiii) 300 mg/m$^2$ per day for 7 days in a 14 day cycle in combination with a growth factor. In some aspects, the dosing regimen is (i) 100 mg/m$^2$ per day for 14 days in a 21 day cycle; (ii) 400 mg/m$^2$ per day for 5 days in a 28 day cycle in combination with a growth factor; (iii) 150 mg/m$^2$ per day for 7 days in a 14 day cycle. When the degree or level of methylation is Moderate, the dosing regimen is (i) 100 mg/m$^2$ per day for 14 days in a 28 day cycle; (ii) 300 mg/m$^2$ per day for 5 days in a 28 day cycle in combination with a growth factor; (iii) 75 mg/m$^2$ per day for 21 days in a 28 day cycle; or (iv) 75 mg/m$^2$ per 42

days in a 56 day cycle. When the degree or level of methylation is High, the dosing regimen is (i) 150-200 mg/m$^2$ per day for 5 days in a 28 day cycle; or (ii) 250 mg/m$^2$ per day for 5 days in a 28 day cycle in combination with a growth factor. In some embodiments, the sample is a tumor biopsy sample. In some embodiments, the MGMT gene is detected using MSP.

In other embodiments, the dosing regimen is based upon the presence or absence of MGMT protein. When the MGMT protein is not detected in a sample obtained from a patient having melanoma, the dosing regimen is 150-200 mg/m$^2$ per day for 5 days in a 28 day cycle. When the MGMT protein is detected, the dosing regimen is selected from (i) 100 mg/m$^2$ per day for 14 days in a 21 day cycle; 150 mg/m$^2$ per day for 7 days in a 14 day cycle; or (iii) 100 mg/m$^2$ per day for 21 days in a 28 day cycle. In some embodiments, the sample is a tumor biopsy sample. In some embodiments, the MGMT protein is detected using Western blot immunoassay, an immunohistochemical technique, or an enzymatic assay for MGMT protein.

In yet other embodiments, the dosing regimen is based upon the level or activity of the MGMT protein detected in a sample. When the level or enzymatic activity of the MGMT protein detected in a sample obtained from the patient is Low, compared to that of normal lymphocytes, the dosing regimen is (i) 150-200 mg/m$^2$ per day for 5 days in a 28 day cycle; or (ii) 250 mg/m$^2$ per day for 5 days in a 28 day cycle in combination with a growth factor. When the level or enzymatic activity of the MGMT protein is Moderate, the dosing regimen is (i) 100 mg/m$^2$ per day for 14 days in a 28 day cycle; (ii) 300 mg/m$^2$ per day for 5 days in a 28 day cycle in combination with a growth factor; (iii) 75 mg/m$^2$ per day for 21 days in a 28 day cycle; or (iv) 75 mg/m$^2$ per 42 days in a 56 day cycle. When the level or enzymatic activity of the MGMT protein detected in a sample obtained from the patient is High, the dosing regimen is selected from (i) 100 mg/m$^2$ per day for 14 days in a 21 day cycle; (ii) 150 mg/m$^2$ per day for 7 days in a 14 day cycle; or (iii) 100 mg/m$^2$ per day for 21 days in a 28 day cycle. In some embodiments, the sample is a tumor biopsy sample.

In yet other embodiments, any of the temozolomide intravenous formulations described herein invention may comprise a manufactured drug product for treating a proliferative disorder. The drug product also comprises product information which comprises instructions for administering the formulation by intravenous infusion over a period of about 0.6 hours to about 2.9 hours or according to any of the dosage regimens described herein, including but not limited to (a) 150-200 mg/m$^2$ per day for 5 days in a 28 day cycle; (b) 250 mg/m$^2$ per day for 5 days in a 28 day cycle in combination with a growth factor; (c) 100 mg/m$^2$ per day for 14 days in a 28 day cycle; (d) 300 mg/m$^2$ per day for 5 days in a 28 day cycle in combination with a growth factor; (e) 75 mg/m$^2$ per day for 21 days in a 28 day cycle; (f) 75 mg/m$^2$ per 42 days in a 56 day cycle; (g) 85 mg/m2 per 21 days in a 28 day cycle; (h) 350 mg/m$^2$ per day for 5 days in a 28 day cycle in combination with a growth factor; (i) 100 mg/m$^2$ per day for 14 days in a 21 day cycle; (j) 400 mg/m$^2$ per day for 5 days in a 28 day cycle in combination with a growth factor; (k) 150 mg/m$^2$ per day for 7 days in a 14 day cycle; (1) 100 mg/m$^2$ per day for 21 days in a 28 day cycle; (m) 150 mg/m$^2$ per day for 14 days in a 28 day cycle; (n) 75 mg/m$^2$ per day daily; (o) 450 mg/m$^2$ per day for 5 days in a 28 day cycle in combination with a growth factor; (p) 150 mg/m$^2$ per day for 14 days in a 21 day cycle; (q) 100 mg/m$^2$ per day daily;(r) 250 mg/m$^2$ per day for 7 days in a 14 day cycle in combination with a growth factor; and (s) 300 mg/m$^2$ per day for 7 days in a 14 day cycle in combination with a growth factor.

Drug products of the invention may be manufactured by combining in a package a desired temozolomide intravenous formulation and product information which comprises instructions for administering the formulation by intravenous infusion over a period of about 0.6 hours to about 2.9 hours or according to any of the dosage regimens described herein.

In order that this invention be more fully understood, the following examples are set forth. These examples are for the purpose of illustration only and are not to be construed as limiting the scope of the invention in any way.

EXAMPLES

**Example 1**

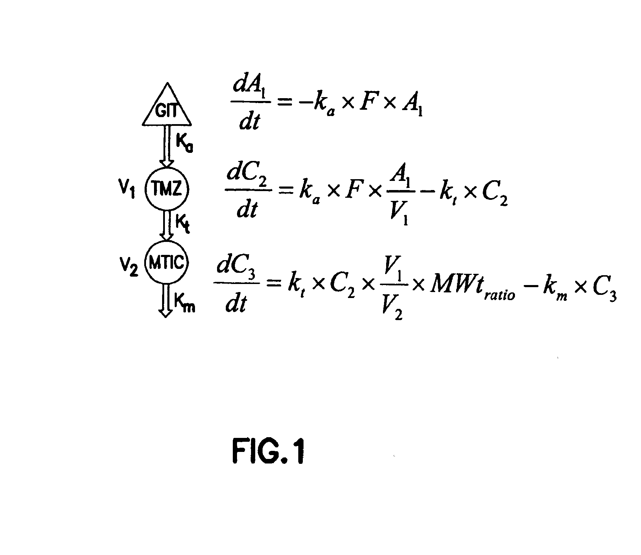

A population pharmacokinetic model was developed to characterize the disposition of both TMZ and MTIC following PO administration. Oral data obtained from a study (see, Middleton, Journal of Clinical Oncology, 15(1):158-166 (2000)) was used to estimate the pharmacokinetic parameters describing the absorption, distribution, metabolism, and excretion of TMZ and MTIC. A PK model with a first order absorption, a one-compartment distribution for TMZ, a first order elimination of TMZ, a one compartment distribution of MTIC, and a first order elimination of MTIC was used. Drugs and Pharmaceutical Sciences, Volume 15: Pharmacokinetics, Gibaldi and Perrier, 2nd Edition1982. The mathematical equations describing the model are shown in Figure 1.

In the equations shown above, $k_a$ is the absorption rate constant, F is the bioavailability of TMZ following oral drug administration, $k_t$ is the rate constant describing the conversion of TMZ to MTIC, $V_1$ is the volume of distribution of TMZ in the human body, $V_2$ is the volume of distribution of MTIC in the human body, $k_m$ is the elimination rate constant of MTIC, MWt$_{ratio}$ is the ratio of the molecular weight of TMZ to that of MTIC, $A_1$ is the amount of TMZ in the gastrointestinal tract (GIT), $C_2$ is the plasma

The oral PK model was confirmed by a posterior checking technique. This technique was performed by simulating the data using a second study [see, Beale et al., Cancer Cehmother. Pharmacol., 44:389-394 (1999)] and comparing the simulated results to the actual results. Table 2 shows the results of this comparison.

**Table 2.**

| Simulated Results vs. Actual Study Results | | | |
|---|---|---|---|
| $n = 12$, Dose $=150mg/m^2$ | | | |
| **TMZ** | **Simulated n=14** | **Actual n=12** | **Simulated n=1400** |
| Mean Cmax | 8.9 | 8.8 | 8.8 |
| s.d. | 1.64 | 4.28 | 1.87 |
| Minimum Cmax | 6.4 | 2.6 | 4.2 |
| Maximum Cmax | 11.6 | 16.8 | 15.6 |
| | | | |
| Mean AUC | 29.0 | 24.8 | 29.8 |
| s.d. | 5.31 | 5.09 | 5.00 |
| Minimum AUC | 20.7 | 17.7 | 17.1 |
| Maximum AUC | 40.5 | 35.4 | 48.2 |
| | | | |
| **MTIC** | **Simulated n=14** | **Actual n=12** | **Simulated n=1400** |
| Mean Cmax | 0.27 | 0.20 | 0.35 |
| s.d. | 0.109 | 0.091 | 0.176 |
| Minimum Cmax | 0.12 | 0.06 | 0.06 |
| Maximum Cmax | 0.52 | 0.38 | 1.41 |
| | | | |
| Mean AUC | 0.91 | 0.59 | 1.09 |
| s.d. | 0.318 | 0.138 | 0.495 |
| Minimum AUC | 0.50 | 0.36 | 0.27 |
| Maximum AUC | 1.40 | 0.80 | 3.85 |

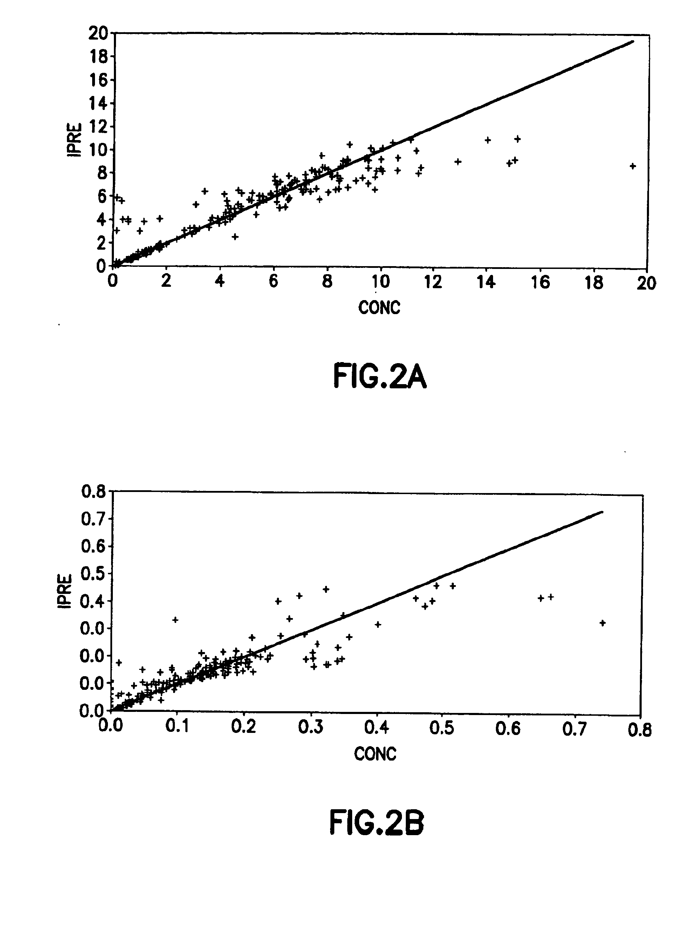

Figure 2 shows a plot of the plasma concentrations for TMZ (Fig. 2A) and MTIC (Fig. 2B) as predicted and observed following oral administration of TMZ.

This comparison showed that that the oral PK model used described well the actual oral PK data obtained from the second study.

Once the oral PK model was verified, it was modified to generate an IV PK model. In particular, the equations from the oral PK model describing the absorption components of TMZ were replaced with equations describing the infusion rate of TMZ. Specifically, the first equation described in Figure 1 was removed and the second equation was replaced with the following equation:

$$\frac{dC_2}{dt} = \frac{K_0}{V_1} - k_t \times C_2 \, ,$$

where $K_0$ is the IV infusion rate. In generating the IV PK model, two assumptions were made. First, the model assumes that there are no first pass effects. That is, once the TMZ is absorbed, it becomes available in the systemic circulation. Second, the model assumes that, since TMZ is infused directly into the systemic circulation, the fraction bioavailable F is equal to one. However, the oral bioavailability (F) of TMZ was unknown.

To validate the IV PK model, the first pass effects and oral bioavailability were determined in a two-way crossover pilot bioavailability study. This was a Phase-1, randomized, open-label pilot study designed to compare the relative bioavailability of IV and PO temozolomide in adult patients with histologically confirmed primary CNS malignancies. The study was conducted in conformance with Good Clinical Practices. Up to 18 patients (to yield 12 evaluable patients) were planned to be enrolled at 1 or 2 centers. 13 patients were enrolled at 2 centers (7 at Center 1, 6 at Center 2). All 13 patients were included in pharmacokinetic and safety analyses. Patients were followed for 23 days after treatment ended for safety evaluations.

After fulfilling all study eligibility requirements, the patients received temozolomide for 5 days (Days 1 to 5) during a 4-week treatment cycle. On Days 1, 2, and 5, patients received temozolomide (200 mg/m$^2$/day) as a PO dose. On Days 3 and 4, patients received Temozolomide (150 mg/m$^2$/day) as a PO dose on one day and as an IV dose on the other day. The IV dose was administered by 1-hour infusion (using an infusion pump) either on Day 3 or Day 4 according to a random code. Pharmacokinetic evaluations were performed on Days 3 and 4 for determination of concentrations of temozolomide and MTIC in plasma. Pharmacokinetic data and the statistical analysis of log-transformed Cmax and AUC are summarized in Table 3.

Table 3: Statistical Analysis of the PK for TMZ and MTIC from pilot study.

| | Subjects | Parameter | Mode of Administration[a] | | Intra subject | Treatment Comparison | Ratio Estimate (%)[c] | 90% C.I. |
| | | | IV Mean[b] | PO Mean[b] | % CV | | | |
|---|---|---|---|---|---|---|---|---|
| MTIC | n=12[d] | AUC (ng.hr/mL)[e] | 607 | 580 | 8 | IV/PO | 104 | 99-111 |
| | | Cmax (ng/mL) | 214 | 179 | 18 | IV/PO | 119 | 105-136 |
| TMZ | n=13 | AUC (ng.hr/mL) | 21.5 | 20.7 | 10 | IV/PO | 104 | 97-112 |
| | | Cmax (ng/mL)[e] | 7.1 | 6.2 | 20 | IV/PO | 114 | 100-131 |

a: The dose of TMZ administered on PK sampling days (both PO and IV) was 150 mg/m2/day.
b: Model-based (least-squares) mean
c: Ratio of the mean value for IV to PO administration
d: the PK date for MTIC from Subject No. 007 for both oral and IV treatments were excluded from the analyses because of improper sample procurement at the study site
e: AUC= AUC(I) for subject profiles with r$^2$≥0.9 and equals AUC(tf) for subject profiles with r$^2$<0.9

This pilot study showed that the oral bioavailability of TMZ was 96 % (range: 89-100%, n=13) in humans.

**Example 2**

Using the IV PK model and Pharsight's Trial Simulator, a clinical trial simulation was performed. Multiple simulation scenarios were performed to evaluate different oral bioavailabilities and the total time with which TMZ would need to be infused into the systemic blood circulation to match the Cmax and AUC obtained following oral administration. Each simulation was repeated 100 times and the probability of success was defined as the number of times the study passed the four bioequivalence criteria, Cmax TMZ, Cmax MTIC, AUC TMZ, and AUC MTIC. The confidence intervals of Cmax and AUC for the IV administration were within 80-125% of those for the oral administration. Table 4 shows the results of these simulations.

**Table 4.**

| | Overall Study Power: Meeting all 4 criteria* | | | |
| --- | --- | --- | --- | --- |
| | Probability of success | | | |
| | 1 hour infusion time | | 1.5 hour infusion time | |
| | F = 0.9 | F = 1.0 | F = 0.9 | F = 1.0 |
| n = 20 | 5 | 52 | 52 | 93 |
| n = 24 | 4 | 73 | 43 | 99 |
| n = 28 | 13 | 82 | 66 | 100 |
| n = 32 | 10 | 88 | 73 | 99 |
| n = 36 | 13 | 89 | 62 | 97 |
| n = 40 | 10 | 93 | 64 | 100 |
| n = 50 | 15 | 96 | 80 | 98 |
| * The four criteria are: Cmax TMZ, Cmax MTIC, AUC TMZ, and AUC MTIC. | | | | |

The simulation results using the IV PK model showed that, if the total oral TMZ bioavailability (F) is greater than or equal to 90% (*i.e.,* F = 0.9-1.0), there will be no need to adjust the IV dose. The simulations also show that the most optimal IV infusion time to match the oral PK profiles of TMZ and MTIC is 1.5 hours.

**Example 3**

An intravenous formulation according to the present invention is provided in Table 5. The formulation was prepared according to the methods of U.S. Patent 6,987,108 and had a pH of about 4.

Table 5.

| Component | Amount (mg/mL) | Function |
| --- | --- | --- |
| Temozolomide | 2.5 | Active Ingredient |
| Polysorbate 80 | 3.0 | Excipient |
| L-Threonine USP | 4.0 | Dissolution enhancing agent |
| Mannitol USP | 15.0 | Bulking agent |
| Sodium Citrate Dihydrate USP | 5.88 | Buffer |
| Hydrochloric Acid NF | 1.48 | pH adjuster |
| Water | q.s. ad 1 mL | Aqueous diluent |

**Example 4**

Twenty-two patients with primary CNS malignancies were enrolled in a clinical bioequivalence study and treated according to the methods of the present invention. Nineteen of the patients were considered to be pharmacokinetic evaluablee. This study was a randomized, open-label, 2-way cross over study of the PK of oral and IV TMZ. The IV formulation of Example 3 is administered at a dosage of 150-200 $mg/m^2$ by intravenous infusion over a period of about 1.5 hours.

The patients are divided into two groups, those receiving Treatment A and those receiving Treatment B. The Treatment A patients receive oral TMZ (200 $mg/m^2$) on Days 1, 2 and 5, IV TMZ 150 mg/m2 on Day 3, and oral TMZ (150 $mg/m^2$) on Day 4 of a 28 day cycle. The Treatment B patients receive oral TMZ (200 $mg/m^2$) on Days 1, 2 and 5, Oral TMZ (150 $mg/m^2$) on Day 3 and IV TMZ (150 $mg/m^2$) on Day 4 of a 28 day cycle. Cmax and AUC for TMZ and MTIC are determined according to methods known to those skilled in the art on Days 3 and 4.

Mean plasma concentration-time data after IV and oral administration for temozolomide and its metabolite MTIC are presented in Tables 6 and 7, respectively.

**Table 6** Mean (CV%) Temozolomide Concentration-Time Data

| | | IV[a] (n=19) | | PO[a] (n=19) |
|---|---|---|---|---|
| Time (hr) | Mean (μg/mL) | CV (%) | Mean (μg/mL) | CV (%) |
| 0 | 0 | 0 | 0 | 0 |
| 0.25 | 2.38[b] | 39 | 2.11 | 129 |
| 0.5 | 3.67 | 30 | 5.32 | 51 |
| 1 | 5.49 | 23 | 6.05 | 34 |
| 1.25 | 6.59[b] | 23 | 6.12[b] | 25 |
| 1.5 | 7.08 | 22 | 5.77 | 24 |
| 1.75 | 6.10 | 23 | 5.36[b] | 25 |
| 2 | 5.60 | 22 | 4.89 | 18 |
| 2.5 | 4.59 | 21 | 4.00 | 19 |
| 3 | 3.86[b] | 18 | 3.44 | 18 |
| 4 | 2.81 | 23 | 2.41 | 19 |
| 6 | 1.20 | 22 | 1.17 | 23 |
| 8 | 0.59 | 26 | 0.56 | 29 |
| IV = intravenous a: The dose of TMZ mg/m$^2$/day. b: n=18 | administration; PO = oral administered on | administration. pharmacokinetic sampling | days (both PO and IV) | was 150 |

**Table 7** Mean (CV%) MTIC Concentration-Time Data

| | IV[a] (n=19) | | PO[a] (n=19) | |
|---|---|---|---|---|
| Time (hr) | Mean (ng/mL) | CV (%) | Mean (ng/mL) | CV (%) |
| 0 | 0 | 0 | 0 | 0 |
| 0.25 | 77.0[b] | 73 | 108 | 187 |
| 0.5 | 144 | 63 | 234 | 94 |
| 1 | 234 | 62 | 247 | 60 |
| 1.25 | 287[b] | 64 | 246[b] | 53 |
| 1.5 | 309 | 62 | 227 | 48 |
| 1.75 | 254 | 59 | 240[b] | 61 |
| 2 | 226 | 48 | 211 | 60 |
| 2.5 | 173 | 50 | 171 | 58 |
| 3 | 155[b] | 47 | 144 | 60 |
| 4 | 105 | 54 | 114 | 64 |
| 6 | 49.7 | 54 | 47.4 | 62 |
| 8 | 23.0 | 69 | 22.6 | 69 |
| IV = intravenous administration; PO = oral administration. a: The dose of TMZ administered on pharmacokinetic sampling days (both PO and IV) was 150 mg/m$^2$/day. b: n=18 | | | | |

The mean plasma pharmacokinetic parameters of temozolomide and MTIC following IV and oral administration of temozolomide are summarized in Table 8.

**Table 8** Mean (CV, %) Pharmacokinetic Parameters of Temozolomide and Its Metabolite MTIC Following Intravenous and Oral Administration of Temozolomide to Subjects With Primary CNS Malignancies

| Analyte | TMZ DOSE* | t½ (hr) | Tmax[b] (hr) | Cmax (ug/mL for TMZ) (ng/mL for MTIC) | tf (hr) | AUC(tf) (ug-hr/mL for TMZ) (ng-hr/mL for MTIC) | AUC(I) (ug-hr/mL for TMZ) (ng-hr/mL for MTIC) |
|---|---|---|---|---|---|---|---|
| TMZ (n=19) | IV | 1.82 (11) | 1.50 (0.92-2.00) | 7.44 (21) | 8.00 (0) | 23.4 (18) | 25.0 (18) |
| | PO | 1.91 (14) | 1.00 (0.25-2.00) | 7.68 (19) | 8.00 (0) | 22.0 (14) | 23.6 (14) |
| MTIC (n=19) | IV | 1.82 (11) | 1.50 (1.25-1.75) | 320 (61) | 8.00 (0) | 941 (53) | 1004 (54) |
| | PO | 1.80 (10) | 1.00 (0.25-2.00) | 333 (62) | 8.00 (0) | 944 (60) | 1004 (60) |

TMZ = temozolomide; MTIC = monoethyl triazenoimidazole carboxamide; PO = oral administration; IV = intravenous administration; t1/2 = terminal-phase half-life; Cmax = maximum observed plasma concentration; Tmax = time to Cmax, tf = time of final quantifiable sample; AUC(tf) = area under the concentration-time curve from 0 hr to time of final quantifiable sample; AUC(I) = area under the concentration-time curve from 0 hour to infinity. a: The dose of TMZ administered on pharmacokinetic sampling days (both PO and IV) was 150 mg/m²/day. b: Median (range)

The treatment ration estimates (IV/PO) and the 90% CIs for the Cmax, AUC(tf), and AUC(I) parameters of temozolomide and its metabolite MTIC are presented in Table 9. Based on statistical comparison of Cmax, AUC(tf), and AUC(I) values of temozolomide and MTIC after IV (1.5-hr infusion) and oral administration of temozolomide for the pharmacokinetic evaluable subjects (n=19), the two formulations are bioequivalent. 90% CIs for temozolomide and MTIC for Cmax, AUC(tf), and AUC(I) are within the accepted bioequivalence guidelines of 80 to 125%.

**Table 9** Relative Bioavailability of Temozolomide and Its Metabolite MTIC Following Intravenous and Oral Administration of Temozolomide to Subjects With Primary CNS Malignancies (Population = PE)

| Assay | Parameter | Mode of Administration[a] | | Ratio Estimate[c] (%) | 90% Confidence Interval |
|---|---|---|---|---|---|
| | | IV Mean[b] | PO Mean[b] | | |
| MTIC (n=19) | Cmax (ng/mL) | 276 | 282 | 98 | 91-105 |
| | AUC(tf) (ng-hr/mL) | 837 | 815 | 103 | 98-108 |
| | AUC(1) (ng·hr/mL) | 891 | 864 | 103 | 98-108 |
| TMZ (n=19) | Cmax (µg/mL) | 7.29 | 7.54 | 97 | 91-102 |
| | AUC(tf) (µg·hr/mL) | 23.1 | 21.8 | 106 | 103-109 |
| | AUC(I) (pg.hr/mL) | 24.6 | 23.4 | 105 | 102-108 |

TMZ = temozolomide; MTIC = monoethyl triazenoimidazole carboxamide; ; AUC(I) = area under the concentration-time curve from 0 hour to infinity; Cmax = maximum observed plasma concentration; PO = oral administration; IV = intravenous administration.
PE: Pharmacokinetic evaluable subjects. Subjects 107, 121, and 122 were excluded.
a: The dose of TMZ administered on pharmacokinetic sampling days (both PO and IV) was 150 mg/m²/day.
b: Model-based (least-squares) mean.
c: Ratio of the mean value for IV to PO administration.

Preferred embodiments of the present invention are described below and are referred to as embodiments E1 to E42.

E1. A method for treating a patient having a proliferative disorder comprising the step of administering to the patient a formulation comprising temozolomide or a pharmaceutically acceptable salt thereof, wherein the formulation is administered by intravenous infusion over a period of about 1 hour to about 2 hours.

E 2. A method for treating a patient having a proliferative disorder comprising the step of administering to the patient a formulation comprising temozolomide or a pharmaceutically acceptable salt thereof by intravenous infusion over a period of about 1 hour to about 2 hours, wherein the administration of a single dose of 150 mg/m2 of the formulation achieves an arithmetic maximum plasma concentration (Cmax) of temozolomide in the range of about 5.5 to about 10.6 $\mu$g/mL and an arithmetic maximum plasma concentration (Cmax) of MTIC in the range of about 137 to about 916 ng/mL.

E 3. The method of E 2, wherein the arithmetic mean maximum plasma concentration (Cmax) of temozolomide is about 7.4 $\mu$g/mL, and the mean maximum plasma concentration (Cmax) of MTIC is about 320 ng/mL.

E 4. A method for treating a patient having a proliferative disorder comprising the step of administering to the patient in need thereof a formulation comprising temozolomide or a pharmaceutically acceptable salt thereof by intravenous infusion over a period of about 1 hour to about 2 hours, wherein a plot of the plasma concentration of temozolomide versus time following the administration of a single dose of 150 mg/m2 of the formulation yields an arithmetic AUC (from time zero to infinity) for temozolomide in the range of about 17.6 to about 37.0 ($\mu$g.hr)/mL, and a plot of the plasma concentration of MTIC versus time yields an arithmetic AUC (from time zero to infinity) for MTIC in the range of about 481 to about 2639 (ng.hr)/mL.

E 5. The method of E 4, wherein the arithmetic mean AUC (from time zero to infinity) for temozolomide is about 25 ($\mu$g.hr)/mL and the arithmetic mean AUC (from time zero to infinity) for MTIC is about 1004 (ng.hr)/mL .

E 6. A method for treating a patient having a proliferative disorder comprising the step of administering to the patient in need thereof a formulation comprising temozolomide or a pharmaceutically acceptable salt thereof by intravenous infusion over a period of about 1 hour to about 2 hours, wherein the administration of a single dose of 150 mg/m2 of the formulation achieves:

an arithmetic maximum plasma concentration (Cmax) of temozolomide in the range of about 5.5 to about 10.6 $\mu$g/mL and an arithmetic maximum plasma concentration (Cmax) of MTIC in the range of about 137 to about 916 ng/mL; and

wherein a plot of the plasma concentration of temozolomide versus time yields an arithmetic AUC (from time zero to infinity) for temozolomide in the range of about 17.6 to about 37.0 ($\mu$g.hr)/mL, and a plot of the plasma concentration of MTIC versus time yields an arithmetic AUC (from time zero to infinity) for MTIC in the range of about 481 to about 2639 (ng.hr)/mL.

E 7. The method of E 6, wherein the arithmetic mean maximum plasma concentration (Cmax) of temozolomide is about 7.4 $\mu$g/mL and the mean maximum plasma concentration (Cmax) of MTIC is about 320 ng/mL; and wherein the arithmetic mean AUC (from time zero to infinity) for temozolomide is about 25 ($\mu$g.hr)/mL and the arithmetic mean AUC (from time zero to infinity) for MTIC is about 1004 (ng.hr)/mL.

E 8. The method of any one of E 1-7, wherein the proliferative disorder is selected from the group consisting of carcinoma, sarcoma, glioma, melanoma, lung cancer, thyroid follicular cancer, pancreatic cancer, anaplastic astrocytoma, pancreatic cancer, bladder cancer, myelodysplasia, prostate cancer, testicular cancer, lymphoma, leukemia, mycosis fungoides, head and neck cancer, breast cancer, ovarian cancer, colorectal and/or colon cancer, and esophageal cancer.

E 9. The method of E 8, wherein the proliferative disorder is glioma.

E 10. The method of E 8, wherein the proliferative disorder is melanoma.

E 11. The method of E 8, wherein the proliferative disorder is lung cancer.

E 12. The method of E 8, wherein the proliferative disorder is non-small cell lung cancer.

E 13. The method of E 8, wherein the proliferative disorder is lymphoma.

E 14. The method of E 8, wherein the proliferative disorder is head and neck cancer.

E 15. The method of E 8, wherein the proliferative disorder is ovarian cancer.

E 16. The method of E 8, wherein the proliferative disorder is colorectal and/or colon cancer.

E 17. The method of E 8, wherein the proliferative disorder is esophageal cancer.

E 18. The method of any one of E 1-17, wherein the formulation is administered by intravenous infusion over a period of about 1.25 hours to about 1.75 hours.

E 19. The method of any one of E 1-17, wherein the formulation is administered by intravenous infusion over a period of about 1.35 hours to about 1.65 hours.

E 20. The method of any one of E 1-17, wherein the formulation is administered by intravenous infusion over a period of about 1.45 hours to about 1.55 hours.

E 21. The method of any one of E1-17, wherein the formulation is administered by intravenous infusion over a period of about 1.5 hours (90 minutes).

E 22. The method of any one of E 1-21, wherein temozolomide or a pharmaceutically acceptable salt thereof is infused intravenously using a pump.

E 23. The method of any one of E 1-22, wherein the formulation further comprises: (a) at least one aqueous diluent; and (b) at least one dissolution enhancing agent sufficient to substantially dissolve the temozolomide, wherein the dissolution enhancing agent is selected from the group consisting urea, L-histidine, L-threonine, L-asparagine, L-serine and L-glutamine.

E 24. The method of any one of E 1-22, wherein the formulation further comprises mannitol, L-threonine, polysorbate-80, sodium citrate dehydrate and hydrochlorid acid.

E 25. The method of E 23 or 24, wherein the formulation is a lyophilized powder.

E 26. The method of E 25, wherein the lyophilized powder is reconstituted in sterile water.

E 27. The method of E26, wherein the formulation reconstituted in sterile water contains 2.5 mg/mL of temozolomide.

E 28. The method of E 25, wherein the lyophilized powder is contained on a vial containing 100 mg of temozolomide, and wherein the lyophilized powder is reconstituted in 41 mL of sterile water.

E 29. The method of any one of E1-28, wherein the formulation is administered at a dose of 75 mg/m2 per day for 42 consecutive days.

E30. The method of E 29, wherein the patient further receives concomitant focal radiotherapy.

E31. The method of E 30, wherein the concomitant focal radiotherapy consists of 60 Gy administered in 30 fractions.

E 32. The method of any one of E 1-28, wherein the formulation is administered at a dose of 150-200 mg/m2 per day for 5 consecutive days in a 28 day cycle.

E 33. The method of any one of E 1-28, wherein the formulation is administered at a dose of 150-200 mg/m2 per day for 7 consecutive days in a 14 day cycle.

E 34. The method of any one of E 1-33, further comprising the step of administering one or more additional therapeutic agents.

E 35. The method of E34, wherein the one or more additional therapeutic agents is administered intravenously.

E 36. A manufactured drug product for treating a proliferative disorder, which comprises:

(a) a pharmaceutical formulation comprising temozolomide or a pharmaceutically acceptable salt thereof, wherein the formulation is in the form of a lyophilized power; and

(b) product information which comprises instructions for administering the pharmaceutical formulation by intravenous infusion over a period of about 1.5 hours (90 minutes).

E 37. The drug product of E 34, wherein the product information further comprises instructions for administering the formulation by intravenous infusion at a dose of 75 mg/m2 per day for 42 consecutive days.

E 38. The drug product of E 35, wherein the product information further comprises instructions for administering concomitant focal radiotherapy.

E 39. The drug product of E36, wherein the concomitant focal radiotherapy consists of 60 Gy administered in 30 fractions.

E 40. The drug product of E34, wherein the product information further comprises instructions for administering the formulation by intravenous infusion at dose of 150-200 mg/m2 per day for 5 consecutive days in a 28 day cycle.

E 41. The drug product of E 34, wherein the product information further comprises instructions for administering the formulation by intravenous infusion at dose of 150-200 mg/m2 per day for 7 consecutive days in a 14 day cycle.

E 42. The method of any one of E 1-22, wherein the method further comprises the oral administration of a formulation comprising temozolomide.

**Claims**

1. Use of temozolomide or a pharmaceutically acceptable salt thereof for the manufacture of a formulation for the treatment of a proliferative disorder in a patient, wherein the treatment comprises administering a dose of 150 mg/m$^2$ of temozolomide to the patient by intravenous infusion over a period of about 1.5 hours (90 minutes), wherein the administration achieves an arithmetic maximum plasma concentration (Cmax) of temozolomide in the range of about 5.5 to about 10.6 μg/mL and an arithmetic maximum plasma concentration (Cmax) of 3-methyl-(triazen-1-yl)imidazole-4-carboxamide (MTIC) in the range of about 137 to about 916 ng/mL; and/or wherein a plot of the plasma concentration of temozolomide versus time as a result of the administration yields an arithmetic AUC (from time zero to infinity) for temozolomide in the range of about 17.6 to about 37.0 ($\mu$g.hr)/mL and a plot of the plasma concentration of MTIC versus time as a result of the administration yields an arithmetic AUC (from time zero to infinity) for MTIC in the range of about 481 to about 2639 (ng.hr)/mL.

2. The use of claim 1, wherein the arithmetic maximum plasma concentration (Cmax) of temozolomide is about 7.4 $\mu$g/mL, and the arithmetic maximum plasma concentration (Cmax) of MTIC is about 320 ng/mL.

3. The use of claim 1 or 2, wherein the arithmetic AUC (from time zero to infinity) for temozolomide is about 25 ($\mu$g.hr) /mL, and the arithmetic AUC (from time zero to infinity) for MTIC is about 1004 (ng.hr)/mL.

4. The use of claim 1, wherein the administration achieves an arithmetic maximum plasma concentration (Cmax) of temozolomide in the range of about 5.5 to about 10.6 $\mu$g/mL and an arithmetic maximum plasma concentration (Cmax) of MTIC in the range of about 137 to about 916 ng/mL; and wherein a plot of the plasma concentration of temozolomide versus time as a result of the administration yields an arithmetic AUC (from time zero to infinity) for temozolomide in the range of about 17.6 to about 37.0 ($\mu$g.hr)/mL and a plot of the plasma concentration of MTIC versus time as a result of the administration yields an arithmetic AUC (from time zero to infinity) for MTIC in the range of about 481 to about 2639 (ng.hr)/mL.

5. The use of any one of claims 1-4, wherein the formulation comprises mannitol, L-threonine, polysorbate-80, sodium citrate dihydrate and hydrochloric acid.

6. The use of any one of claims 1-5, wherein the proliferative disorder is glioma.

7. The use of any one of claims 1-6, wherein the formulation is a lyophilized powder that is reconstituted prior to its administration to the patient.

8. The use of claim 7, wherein the lyophilized powder is reconstituted in sterile water.

9. The use of claim 7 or 8, wherein the reconstituted formulation contains 2.5 mg/mL of temozolomide.

10. Temozolomide or a pharmaceutically acceptable salt thereof, for use in the treatment of a proliferative disorder in a patient, wherein the treatment comprises administering a dose of 150 mg/m$^2$ oftemozolomide to the patient by intravenous infusion over a period of about 1.5 hours (90 minutes), wherein the administration achieves an arithmetic maximum plasma concentration (Cmax) of temozolomide in the range of about 5.5 to about 10.6 $\mu$g/mL and an arithmetic maximum plasma concentration (Cmax) of MTIC in the range of about 137 to about 916 ng/mL; and/or wherein a plot of the plasma concentration of temozolomide versus time as a result of the administration yields an arithmetic AUC (from time zero to infinity) for temozolomide in the range of about 17.6 to about 37.0 ($\mu$g.hr)/mL and a plot of the plasma concentration of MTIC versus time as a result of the administration yields an arithmetic AUC (from time zero to infinity) for MTIC in the range of about 481 to about 2639 (ng.hr)/mL.

11. The temozolomide or pharmaceutically acceptable salt thereof of claim 10, for the use as defined in claim 10, wherein the administration achieves an arithmetic maximum plasma concentration (Cmax) of temozolomide in the range of about 5.5 to about 10.6 $\mu$g/mL and an arithmetic maximum plasma concentration (Cmax) of MTIC in the range of about 137 to about 916 ng/mL; and wherein a plot of the plasma concentration of temozolomide versus time as a result of the administration yields an arithmetic AUC (from time zero to infinity) for temozolomide in the range of about 17.6 to about 37.0 ($\mu$g.hr)/mL and a plot of the plasma concentration of MTIC versus time as a result of the administration yields an arithmetic AUC (from time zero to infinity) for MTIC in the range of about 481 to about 2639 (ng.hr)/mL.

12. The temozolomide or pharmaceutically acceptable salt thereof of claim 10, for the use as defined in claim 10, wherein the arithmetic maximum plasma concentration (Cmax) of temozolomide is about 7.4 $\mu$g/mL, and the arithmetic maximum plasma concentration (Cmax) of MTIC is about 320 ng/mL.

13. The temozolomide or pharmaceutically acceptable salt thereof of claim 10 or claim 12, for the use as defined in claim 10, wherein the arithmetic AUC (from time zero to infinity) for temozolomide is about 25 ($\mu$g.hr)/mL, and the arithmetic AUC (from time zero to infinity) for MTIC is about 1004 (ng.hr)/mL.

14. The temozolomide or pharmaceutically acceptable salt thereof of any one of claims 10-13, for the use as defined in claim 10, wherein the temozolomide or pharmaceutically acceptable salt thereof is in a formulation comprising mannitol, L-threonine, polysorbate-80, sodium citrate dihydrate and hydrochloric acid.

15. The temozolomide or pharmaceutically acceptable salt thereof of claim 14, for the use as defined in claim 10, wherein the formulation is a lyophilized powder that is reconstituted prior to its administration to the patient.

16. The temozolomide or pharmaceutically acceptable salt thereof of claim 15, for the use as defined in claim 10, wherein the lyophilized powder is reconstituted in sterile water.

17. The temozolomide or pharmaceutically acceptable salt thereof of claim 15 or 16, for the use as defined in claim 10, wherein the reconstituted formulation contains 2.5 mg/mL of temozolomide.

18. The temozolomide or pharmaceutically acceptable salt thereof of any one of claims 10-17, for the use as defined in claim 10, wherein the proliferative disorder is glioma.

$$\frac{dA_1}{dt} = -k_a \times F \times A_1$$

$$\frac{dC_2}{dt} = k_a \times F \times \frac{A_1}{V_1} - k_t \times C_2$$

$$\frac{dC_3}{dt} = k_t \times C_2 \times \frac{V_1}{V_2} \times MWt_{ratio} - k_m \times C_3$$

# FIG.1

FIG.2A

FIG.2B

**EUROPEAN SEARCH REPORT**

Application Number

EP 11 18 5843

Europäisches Patentamt
European Patent Office
Office européen des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MARZOLINI CATIA ET AL: "Pharmacokinetics of temozolomide in association with fotemustine in malignant melanoma and malignant glioma patients: Comparison of oral, intravenous, and hepatic intra-arterial administration", CANCER CHEMOTHERAPY AND PHARMACOLOGY, vol. 42, no. 6, November 1998 (1998-11), pages 433-440, XP002491149, ISSN: 0344-5704 * table 1 * | 1-18 | INV. A61K31/4188 A61P35/00 |
| X | US 2004/043001 A1 (UGWU SYDNEY [US] ET AL) 4 March 2004 (2004-03-04) * paragraphs [0052] - [0054] * | 1-18 | |
| Y | US 2006/100188 A1 (ZONG CHEN [US] ET AL) 11 May 2006 (2006-05-11) * claims 1,11,31,41,51,61 * | 1-18 | |

TECHNICAL FIELDS SEARCHED (IPC)

A61K
A61P

The present search report has been drawn up for all claims

| Place of search | Date of completion of the search | Examiner |
|---|---|---|
| Munich | 25 November 2011 | Loher, Florian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

## ANNEX TO THE EUROPEAN SEARCH REPORT
## ON EUROPEAN PATENT APPLICATION NO.

EP 11 18 5843

This annex lists the patent family members relating to the patent documents cited in  the above-mentioned European search report.
The members are as contained in the European Patent Office EDP file on
The European Patent Office is in no way liable for these particulars which are merely given for the purpose of information.

25-11-2011

| Patent document cited in search report | Publication date | Patent family member(s) | Publication date |
|---|---|---|---|
| US 2004043001 A1 | 04-03-2004 | US 2004043001 A1<br>US 2006122163 A1<br>US 2009176852 A1<br>US 2010331382 A1 | 04-03-2004<br>08-06-2006<br>09-07-2009<br>30-12-2010 |
| US 2006100188 A1 | 11-05-2006 | US 2006100188 A1<br>US 2009247598 A1 | 11-05-2006<br>01-10-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5260291 A
- US 6987108 B
- WO 0206284 A, Novartis
- WO 0206247 A, Bayer
- US 6703400 B
- US 7119200 B
- US 7161003 B
- US 7166602 B
- US 7196078 B
- US 6107305 A
- WO 0210162 A
- WO 016465 A
- US 6413755 B
- US 6383744 B
- US 6211164 B
- WO 0116306 A
- WO 0121771 A
- WO 0016781 A
- WO 02070494 A
- WO 2007044449 A
- WO 2007044441 A
- WO 2007044426 A
- WO 2007044420 A
- WO 2007044410 A
- WO 2007044407 A
- WO 2007044401 A
- WO 2007041712 A
- US 5824346 A
- US 5939098 A
- US 5942247 A
- US 6096757 A
- US 6251886 B
- US 6316462 B
- US 6333333 B
- US 6346524 B
- US 20060100188 A
- US 5786146 A
- US 6017704 A
- US 6200756 B
- US 6265171 B
- US 6773897 B
- US 5817514 A, Li
- US 5407804 A
- US 6331393 B

### Non-patent literature cited in the description

- **STUPP et al.** *J. Clin. Onc.,* 2002, vol. 20 (5), 1375-1382
- **BAKER.** *Clinical Cancer Research,* 1999, vol. 5, 309-317
- **LEE et al.** *Proc. Natl. Acad. Sci. U.S.A.,* 1985, vol. 82, 4360
- *Journal of Pharmaceutical Science,* 1977, vol. 66, 2
- **MIKNYOCZKI et al.** *Mol Cancer Ther,* 2003, vol. 2 (4), 371-382
- **LIAUDET et al.** *Br J Pharmacol,* 2001, vol. 133 (8), 1424-1430
- **ABDELKARIM et al.** *Int J Mol Med,* 2001, vol. 7 (3), 255-260
- **MABLEY et al.** *Br J Pharmacol,* 2001, vol. 133 (6), 909-919
- **TENTORI et al.** *Int J Oncol,* 2005, vol. 26 (2), 415-422
- **WALISSER ; THIES.** *Exp Cell Res,* 1999, vol. 251 (2), 401-413
- **DI PAOLA et al.** *Eur J Pharmacol,* 2004, vol. 492 (2-3), 203-210
- **BRYANT ; HELLEDAY.** *Biochem Soc Trans,* 2004, vol. 32, 959-961
- **VEUGER et al.** *Cancer Res,* 2003, vol. 63 (18), 6008-6015
- **VEUGER et al.** *Oncogene,* 2004, vol. 23 (44), 7322-7329
- **FARMER et al.** Targeting the DNA repair defect in BRCA mutant cells as a therapeutic strategy. *Nature,* 2005, vol. 434 (7035), 917-921
- **JAGTAP et al.** *Crit Care Med,* 2002, vol. 30 (5), 1071-1082
- **LOH et al.** *Bioorg Med Chem Lett,* 2005, vol. 15 (9), 2235-2238
- **FERRARIS et al.** *J Med Chem,* 2003, vol. 46 (14), 3138-3151
- **FERRARIS et al.** *Bioorg Med Chem Lett,* 2003, vol. 13 (15), 2513-2518
- **FERRARIS et al.** *Bioorg Med Chem,* 2003, vol. 11 (17), 3695-3707
- **LI ; ZHANG.** *IDrugs,* 2001, vol. 4 (7), 804-812
- **STEINHAGEN et al.** *Bioorg Med Chem Lett,* 2002, vol. 12 (21), 3187-3190

- **K. S. KIM et al.** *J. Med. Chem.,* 2002, vol. 45, 3905-3927
- **SANCHEZ, Y.** *Science,* 1997, vol. 277, 1497-1501
- **FLAGGS, G.** *Current Biology,* 1997, vol. 7, 977-986
- **AHRENDT et al.** *J. Natl. Cancer Inst.,* 1999, vol. 91, 332-339
- **BELSINKY et al.** *Proc. Natl. Acad. Sci. U.S.A.,* 1998, vol. 95, 11891-11896
- **CLARK et al.** *Nucleic Acids Res.,* 1994, vol. 22, 2990-2997
- **HERMAN et al.** *Proc Natl Acad Sci U.S.A.,* 1996, vol. 93, 9821-9826
- **XIONG ; LAIRD.** *Nucleic Acids Res.,* 1997, vol. 25, 2532-2534
- **EADS et al.** *Nuc. Acids. Res.,* 2004, vol. 28, e32
- **COTTRELL et al.** *Nucleic Acids Res.,* 2004, vol. 32, 1-8
- **HERMAN et al.** *Proc. Natl. Acad Sci. USA,* 1996, vol. 93 (18), 9821-9826
- **ESTELLER et al.** *Cancer Res.,* 1999, vol. 59, 793-797
- **MYRNES et al.** *Carcinogenesis,* 1984, vol. 5, 1061-1064
- **FUTSCHER et al.** *Cancer Comm.,* 1989, vol. 1, 65-73
- **KREKLAW et al.** *J. Pharmacol. Exper. Ther.,* 2001, vol. 297 (2), 524-530
- **NAGEL et al.** *Anal. Biochem.,* 2003, vol. 321 (1), 38-43
- **XIONG et al.** *Nuc. Acids Res.,* 1997, vol. 25, 2532-2534
- **COSTELLO et al.** *J. Biol. Chem.,* 1994, vol. 269 (25), 17228-17237
- **QIAN et al.** *Carcinogen,* 1995, vol. 16 (6), 1385-1390
- **TANO et al.** *Proc. Natl. Acad. Sci. USA,* 1990, vol. 87, 686-690
- **WATTS et al.** *Mol. Cell. Biol.,* 1997, vol. 17 (9), 5612-5619
- **MIDDLETON.** *Journal of Clinical Oncology,* 2000, vol. 15 (1), 158-166
- **GIBALDI ; PERRIER.** Drugs and Pharmaceutical Sciences, Volume 15: Pharmacokinetics. 1982, vol. 15
- **BEALE et al.** *Cancer Cehmother. Pharmacol.,* 1999, vol. 44, 389-394